# EUROPÄISCHE PATENTSCHRIFT

(11) **EP 0 672 701 B1**
(45) Veröffentlichungstag und Bekanntmachung des Hinweises auf die Patenterteilung: **28.07.1999**
(21) Anmeldenummer: 95103319.0
(22) Anmeldetag: 08.03.1995
(51) Int. Cl.: C08G 69/06, C07D 473/18, C07D 473/34, C07D 239/54, C07D 239/46, C08G 69/10

(54) **PNA-Synthese unter Verwendung einer basenlabilen Amino-Schutzgruppe**
PNA-synthesis using a base labile aminoprotecting group
Synthèse de PNA en utilisant un groupe aminoprotecteur labile en milieu basique

(30) Priorität: 14.03.1994 DE 4408533
(43) Veröffentlichungstag der Anmeldung: 20.09.1995
(73) Patentinhaber: HOECHST AKTIENGESELLSCHAFT, 65929 Frankfurt am Main (DE)
(72) Erfinder: Breipohl, Gerhard, Dr., D-60529 Frankfurt (DE); Uhlmann, Eugen, Dr., D-61479 Glashütten (DE); Knolle, Jochen, Dr., D-65830 Kriftel (DE)

(56) Entgegenhaltungen:
- WO-A-92/20702
- WO-A-93/12129
- ANGEWANDTE CHEMIE, Bd.31, Nr.8, 1992 Seiten 1008 - 1010 CHRIS MEIER ET AL
- JOURNAL OF THE AMERICAN CHEMICAL SOCIETY, Bd.114, Nr.5, 26. Februar 1992 Seiten 1895 - 1897 MICHAEL EGHOLM ET AL
- JOURNAL OF THE AMERICAN CHEMICAL SOCIETY, Bd.114, Nr.24, 18. November 1992 Seiten 9677 - 9678 MICHAEL EGHOLM ET AL
- THE JOURNAL OF ORGANIC CHEMISTRY, Bd.59, Nr.19, 23. September 1994 Seiten 5767 - 5773 KIM L. DUEHOLM ET AL

## Beschreibung

Peptide Nucleic Acids (PNA) sind DNA-analoge Verbindungen in denen das Deoxyribose-Phosphat-Gerüst durch ein Peptid-Oligomer ersetzt wurde. Die bisher in der Literatur (Michael Egholm, Peter E. Nielsen, Ole Buchardt and Rolf H. Berg, J. Am. Chem. Soc. 1992, 114, 9677-9678; Ole Buchardt, Michael Egholm, Peter E. Nielsen and Rolf H. Berg, WO 92/20702) beschriebenen Synthesen verwenden zum temporären Schutz der Aminogruppe des Monomers die säurelabile tert.-Butyloxycarbonyl (Boc)-Schutzgruppe, die durch mittelstarke Säuren, wie z.B. Trifluoressigsäure abgespalten wird. Die Festphasensynthese von Oligomeren folgt dabei dem üblichen Peptidsyntheseverfahren, wie es z.B. von Merrifield (B. Merrifield, J. Am. Chem. Soc., 1963, 85, 2149) beschrieben wurde. Die Abspaltung des PNA-Oligomeren vom festen Träger erfolgt dabei mit einer starken Säure, üblicherweise mit flüssigem Fluorwasserstoff.
Diese Reaktionsbedingungen, insbesondere die wiederholte Behandlung mit Trifluoressigsäure läßt daher eine Reaktion in offenen Reaktiongefäßen nicht zu, wie es zum Beispiel beim Einsatz von multiplen Peptidsynthesizern der Fall ist (Review: G. Jung und A. Beck-Sickinger, Angew. Chem. 104 (1992) 375-391).

Ziel der Erfindung ist, ein Syntheseverfahren mit einer basenlabilen temporären Amino-Schutzgruppe für den Aufbau der PNA-Oligomeren zu entwickeln, das eine Abspaltung vom festen Träger unter schwachen oder mittelstarken Säuren erlaubt.

Die nachfolgende Erfindung beschreibt ein Verfahren zur Herstellung von PNA-Oligomeren der Formel I 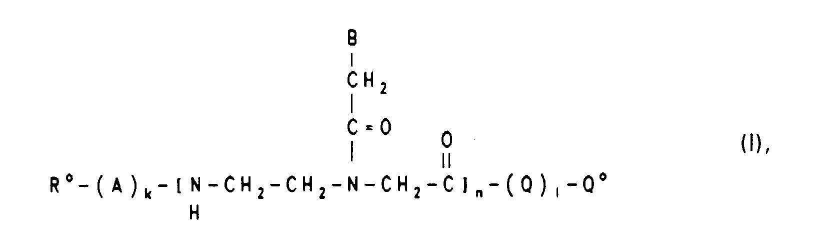 worin
- R^{o}: Wasserstoff, C₁-C₁₈-Alkanoyl, C₁-C₁₈-Alkoxycarbonyl, C₃-C₈-Cycloalkanoyl, C₇-C₁₅-Aroyl, C₃-C₁₃-Heteroaroyl, oder eine Gruppe bedeuten, die die intrazelluläre Aufnahme des Oligomers begünstigt oder die bei der Hybridisierung mit der target-Nucleinsäure interagiert;
- A: ein Aminosäurerest, bevorzugt aus der Reihe Glycin, Leucin, Histidin, Phenylalanin, Cystein, Lysin, Arginin, Asparaginsäure, Glutaminsäure, Prolin, Tetrahydrochinolin-3-carbonsäure, Octahydroindol-2-carbonsäure und N-(2-Aminoethyl)glycin;
- k: eine ganze Zahl von Null bis 20, bevorzugt Null bis 10;
- Q: ein Aminosäurerest, bevorzugt aus der Reihe Glycin, Leucin, Histidin, Phenylalanin, Cystein, Lysin, Arginin, Asparaginsäure, Glutaminsäure, Prolin, Tetrahydrochinolin-3-carbonsäure, Octahydroindol-2-carbonsäure und N-(2-Aminoethyl)glycin;
- I: eine ganze Zahl von Null bis 20, bevorzugt Null bis 10;
- B: für eine in der Nucleotidchemie übliche Nucleobase, beispielsweise für natürliche Nucleobasen wie Adenin, Cytosin, Guanin, Thymin und Uracil oder nicht-natürliche Nucleobasen wie Purin, 2.6-Diaminopurin, 7-Deazaadenin, 7-Deazaguanin, N⁴N⁴-Ethanocytosin, N⁶N⁶-Ethano-2.6-diaminopurin, 5-Methylcytosin, 5-(C₃-C₆)-Alkinyl-uracil, 5-(C₃-C₆)-Alkinylcytosin, 5-Fluor-uracil oder Pseudoisocytosin, 2-Hydroxy-5-methyl-4-triazolopyrimidin oder deren Prodrugformen;
- Q^{o}: Hydroxy, NH₂, NHR" bedeutet mit R" = C₁-C₁₈-Alkyl, C₂-C₁₈-Aminoalkyl, C₂-C₁₈-Hydroxyalkyl; und
- n: eine ganze Zahl von 1- 50, bevorzugt 4-35 bedeuten,
das dadurch gekennzeichnet ist, daß man auf einen polymeren Träger der Formel II

L-[Polymer] (II),

der mit einer Ankergruppe L versehen ist, die latent den Rest Q^{o} enthält, entweder zuerst mit einem für die Festphasensynthese üblichen Verfahren Aminosäuren (Q') aufkuppelt und dann auf die dabei als Zwischenprodukt entstehende Verbindung der Formel III

(Q')ₗ-L-[Polymer] (III),

worin L wie oben definiert ist, Q' für eine Aminosäure Q steht, bei der die gegebenenfalls vorhandenen Seitenkettenfunktionen geschützt sind, und I für eine ganze Zahl von Null bis 20 steht,
oder direkt auf den polymeren Träger der Formel II
a) eine Verbindung der Formel IV 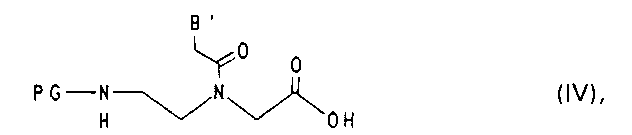 worin
   - PG: für eine basenlabile Aminoschutzgruppe und
   - B': für eine Nucleobase stehen deren exocyclische Aminofunktion durch eine mit der basenlabilen Aminoschutzgruppe Kompatiblen Schutzgruppe geschützt ist unter Verwendung der in der Peptidchemie üblichen Kupplungsreagenzien aufkuppelt,
b) die temporäre basenlabile Schutzgruppe PG mittels eines geeigneten Reagenzes abspaltet,
c) die Schritte a und b (n-1)fach wiederholt,
d) mit einem für die Festphasensynthese üblichen Verfahren weitere Aminosäuren A', die wie A definiert sind, jedoch in der Seitenkette gegebenfalls geschützt sind, aufkuppelt und dann für den Fall, daß R^{o} nicht Wasserstoff ist, den Rest R^{o} mit einem üblichen Verfahren einführt,
e) aus der als Zwischenverbindung erhaltenen Verbindung der Formel Ia 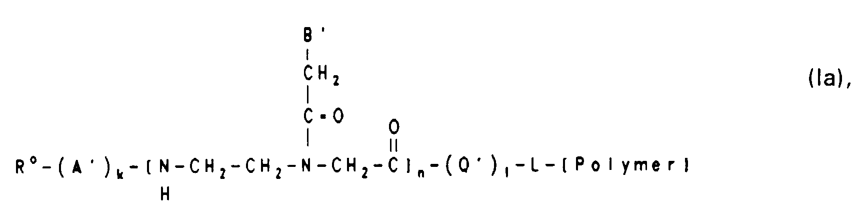 worin R^{o}, A', k, B', n, Q' und I wie oben definiert sind und L für eine Ankergruppe steht, die Verbindung der Formel I mittels eines Abspaltungsreagenzes vom polymeren Träger abspaltet wobei gleichzeitig oder auch anschließend die an der exocyclischen Aminofunktion der Nucleobasen und an den Seitenketten der Aminosäuren gegebenenfalls vorhandenen Schutzgruppen abgespalten werden.

Gruppen, die die intrazelluläre Aufnahme des Oligomers begünstigen sind zum Beispiel Alkanoyl- und Alkoxycarbonylverbindungen mit verschiedenen lipophilen Resten wie -(CH₂)ₓ-CH₃, worin x eine ganze Zahl von 6-18 bedeutet, -(CH₂)ₙ-CH = CH-(CH₂)ₘ-CH₃, worin n und m unabhängig voneinander eine ganze Zahl von 6 bis 12 bedeuten, -(CH₂CH₂O)₄-(CH₂)₉-CH₃, -(CH₂CH₂O)₈ - (CH₂)₁₃-CH₃ und -(CH₂CH₂O)₇-(CH₂)₁₅-CH₃, aber auch Steroid-Reste wie Cholesteryl oder Vitamin-Reste wie Vitamin E, Vitamin A oder Vitamin D und andere Konjugate, die natürliche Carriersysteme ausnutzen wie Gallensäure, Folsäure, 2-(N-Alkyl, N-Alkoxy)-Aminoanthrachinon und Konjugate der Mannose und Peptide der entsprechenden Rezeptoren, die zur rezeptorvermittelten Endozytose der Oligomeren führen wie EGF (Epidermal Growth Factor), Bradykinin und PDGF (Platelet Derived Growth Factor). Unter Markierungs-Gruppen sind fluoreszierende Gruppen beispielsweise von Dansyl-(N-Dimethyl-1-aminonaphthyl-5-sulfonyl-), Fluorescein- oder Coumarin-Derivaten oder chemilumineszierende Gruppen beispielsweise von Acridin-Derivaten zu verstehen sowie das über ELISA nachweisbare Digoxygenin-System, die über das Biotin/Avidin-System nachweisbare Biotin-Gruppe oder aber Linker-Arme mit funktionellen Gruppen, die eine nachträgliche Derivatisierung mit nachweisbaren Reporter-Gruppen gestatten, beispielsweise ein Aminoalkyl-Linker, der mit einem Acridinium-Aktivester zur Chemilumineszenz-Probe umgesetzt wird. Typische Markierungsgruppen sind:

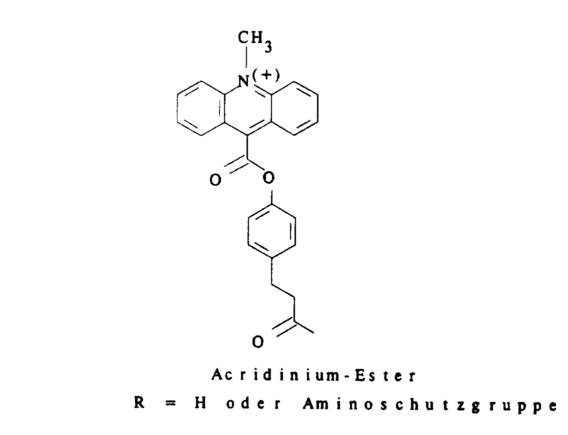

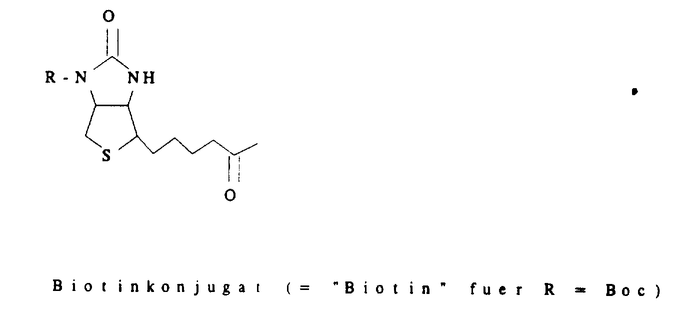

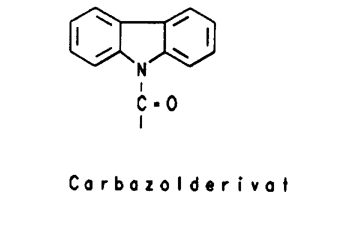

Gruppen, die bei der Hybridisierung des Oligomers an die target-Nucleinsäure diese unter Bindung, Quervernetzung oder Spaltung angreifen sind zum Beispiel Acridin-, Psoralen-, Phenanthridin, Naphtochinon-, Daunomycin- oder Chlorethylaminoaryl-Konjugate. Typische interkalierende und quervernetzende Reste sind:

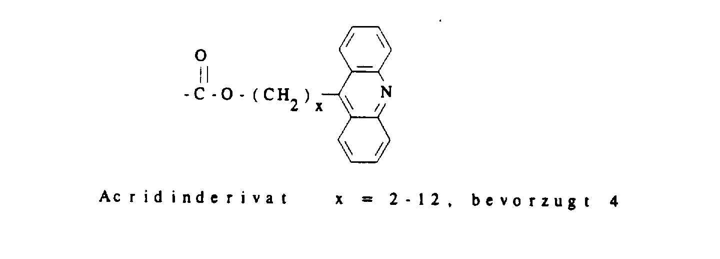

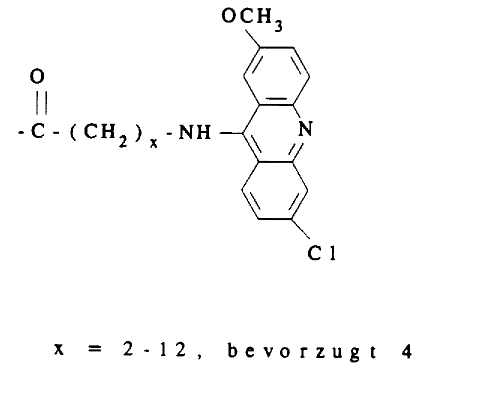

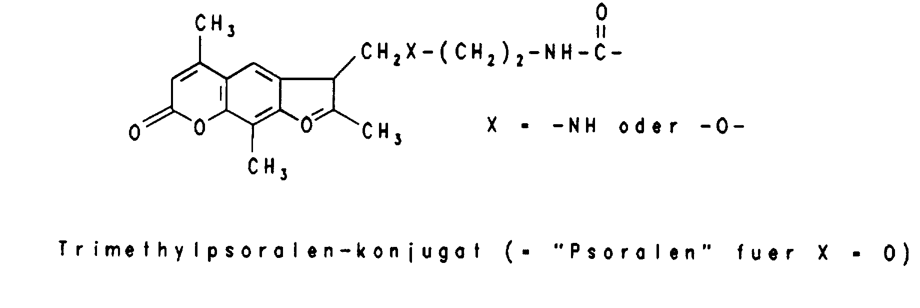

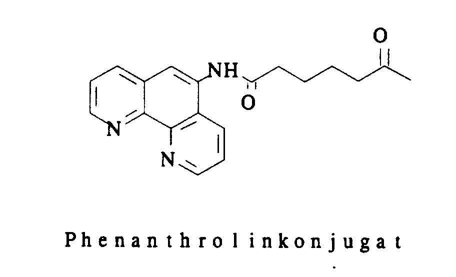

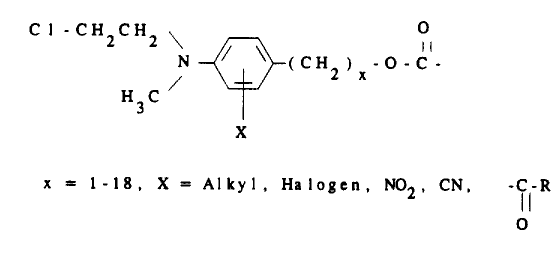

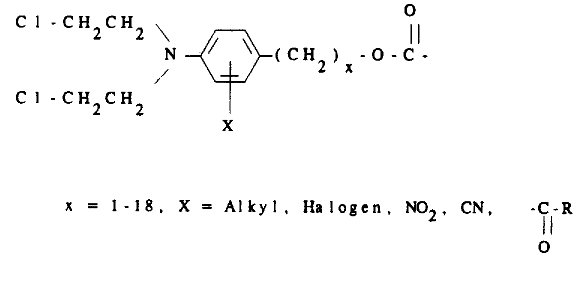

Ankergruppen L, die latent die Funktion Q^{o} enthalten sind zum Beispiel bei George Barany, Nancy Kneib-Cordonier and Daniel G. Mullen., Int. J. Peptide Protein Res., 1987, 30, 705-739; Gregg B. Fields and Richard L. Noble, Int. J. Peptide Protein Res. 35, 1990, 161-214; K. Barlos, D. Gatos, J. Hondrelis, J. Matsoukas, G.J. Moore, W. Schäfer und P. Sotiriou, Liebigs Ann. Chem. 1989, 951-955; H. Rink, Tetrahedron Lett. 1987, 3787-3790; G. Breipohl, J. Knolle und R. Geiger, Tetrahedron Lett. 1987, 5647-5650; G. Breipohl, J. Knolle und W. Stüber, Int. J. Peptide Protein Res., 1989, 34, 262-267; W. Stüber, J. Knolle und G. Breipohl, Int. J. Peptide Protein Res., 1989, 34, 215-220 oder in den EP-A-O 264 802 (HOE 86/F259), EP-A-O 287 882 (HOE 86/F101), EP-A-O 322 348 (HOE 87/F386K) beschrieben.

Polymere Träger, die mit einer Ankergruppe versehen sind, die latent die Gruppe Q^{o} enthalten, sind beispielsweise 4-Alkoxybenzylalkohol-Harz, 2-Methoxy-4-alkoxybenzylalkohol-Harz, 2-Chlortriphenylmethyl-Harz, 2,4-Dimethoxybenzhydrylamin-Harz, 4-(2',4'-Dimethoxyphenylaminomethyl) phenoxymethyl-Harz, oder die mit einer primären Aminogruppe funktionalisierten polymeren Träger, wie z.B. ®polyHIPE, ®Tentagel, ®Controlled Pore Glass, Polystyrol, auf die eine der latent die Gruppe Q^{o} enthaltende Ankergruppen wie z.B. 4-(4'-Methoxybenzhydryl)-phenoxyessigsäure, 4-(4'-Methoxybenzhydryl)-phenoxybuttersäure, 4-Hydroxmethylphenoxyessigsäure, 2-Methoxy-4-hydroxymethylphenoxyessigsäure, 5-(4-Aminomethyl-3,5-dimethoxyphenoxy)-valeriansäure, 3-(Amino-4-methoxybenzyl)-4-methoxyphenylpropionsäure, 5-(Amino-4-methoxybenzyl)-2,4-dimethoxyphenylpropionsäure, 4-(Amino-(C₂-C₈)alkylaminocarbonyloxymethyl)phenoxyessigsäure, Oxalsäuremono(amino-C₂-C₁₆-alkyl)ester, Bernsteinsäuremono(amino-C₂-C₁₆-alkyl)ester aufgekuppelt ist.

Bevorzugt verwendet werden die folgenden Ankergruppen oder schon an den polymeren Träger gebundenen Ankergruppen:
4-Alkoxybenzylalkohol-Harz, Methoxy-4-alkoxybenzylalkohol-Harz, 2-Chlortriphenylmethyl-Harz, oder die auf mit einer primären Aminogruppe funktionalisierten Träger vom Typ ®Tentagel, ®Controlled Pore Glass, Polystyrol, latent die Gruppe Q^{o} enthaltenden gekuppelten Ankergruppen 4-(4'-Methoxybenzhydryl)-phenoxybuttersäure, 4-Hydroxmethylphenoxyessigsäure, 2-Methoxy-4-hydroxmethylphenoxyessigsäure, 5-(Amino-4-methoxybenzyl)-2,4-dimethoxyphenylpropionsäure, 4-(Amino-(C₂-C₈)-alkylaminocarbonyloxymethyl)phenoxyessigsäure, Oxalsäuremono(amino-C₂-C₁₆-alkyl)ester, Bernsteinsäuremono(amino-C₂-C₁₆-alkyl)ester.

Basenlabile Aminoschutzgruppen PG sind z.B. 9-Fluorenylmethoxycarbonyl (Fmoc) und 2,2-[Bis(4-nitrophenyl)]-ethoxycarbonyl (Bnpeoc) (W. König, D. Bücher, R. Knüttel, K. Lindner und A. Volk, Proceedings of the Akabori Conference, Grainau-Eibsee/Bavaria, June 12-13, 1985, pp.32 und R. Ramage, A.J. Blake, M.R. Florence, Th. Gray, G. Raphy, und P.L. Roach, Tetrahedron 1991, 37, 8001-8024), 2-(2,4-Dinitrophenyl)-ethoxycarbonyl (Dnpeoc) (M. Acedo, F. Albericio, R. Eritja, Tetrahedron Lett. 1992, 4989-4992), 2-Methylsulfonylethyloxycarbonyl (Msc), 1'-(4,4-Dimethyl-2,6-dioxocyclohexyliden)ethyl (Dde) (B.W. Bycroft, W.C. Chan, S.R. Chabra und N.D. Home, J.Chem.Soc., Chem. Commun. 1993, 778-779), bevorzugt ist die Verwendung von Fmoc, Bnpeoc und Dnpeoc, ganz besonders bevorzugt wird die Fmoc-Schutzgruppe verwendet.

Die in Schritt a des obigen Syntheseverfahrens benutzten in der Peptidsynthese üblichen Aktivierungsmethoden sind z. B. in Houben-Weyl, Methoden der organischen Chemie, Band 15/2, Georg Thieme Verlag Stuttgart 1974, beschrieben. Weitere Reagenzien wie z.B. BOP (B. Castro, J.R. Dormoy, G. Evin and C. Selve, Tetrahedron Lett. 1975, 1219-1222), PyBOP (J. Coste, D. Le-Nguyen und B. Castro, Tetrahedron Lett. 1990, 205-208), BroP (J. Coste, M.-N. Dufour, A. Pantaloni und B. Castro, Tetrahedron Lett. 1990, 669-672), PyBroP (J. Coste, E. Frerot, P. Jouin und B. Castro, Tetrahedron Lett. 1991, 1967-1970) und Uronium-Reagenzien, wie z.B. HBTU (V. Dourtoglou, B. Gross, V. Lambropoulou, C. Zioudrou, Synthesis 1984, 572-574), TBTU, TPTU, TSTU, TNTU (R. Knorr, A. Trzeciak, W. Bannwarth and D. Gillessen, Tetrahedron Letters 1989, 1927-1930), TOTU (EP-A-0 460 446), HATU (L.A. Carpino, J. Am. Chem. Soc. 1993, 115, 4397-4398), HAPyU, TAPipU (A. Ehrlich, S. Rothemund, M. Brudel, M. Beyermann, L.A. Carpino und M. Bienert, Tetrahedron Lett. 1993, 4781-4784), BOI (K. Akaji, N. Kuriyama, T. Kimura, Y. Fujiwara und Y. Kiso, Tetrahedron Lett. 1992, 3177-3180) oder Säurechloride bzw. Säurefluoride (L. A. Carpino, H. G. Chao, M. Beyermann and M. Bienert, J. Org. Chem., 56 (1991), 2635; J.-N. Bertho, A. Loffet, C. Pinel, F. Reuther and G. Sennyey in E. Giralt and D. Andreu (Eds.) Peptides 1990, Escom Science Publishers B. V. 1991, pp. 53-54; J. Green und K. Bradley, Tetrahedron 1993, 4141-4146), 2,4,6-Mesitylensulfonyl-3-nitro-1,2,4-triazolid (MSNT) (B. Blankemeyer-Menge, M. Nimitz und R. Frank, Tetrahedron Lett. 1990, 1701-1704), 2,5-Diphenyl-2,3-dihydro-3-oxo-4-hydroxythiophendioxid (TDO) (R. Kirstgen, R.C. Sheppard, W. Steglich, J. Chem. Soc. Chem. Commun. 1987, 1870-1871) oder aktivierte Ester (D. Hudson)Peptide Res. 1990, 51-55) sind in den jeweiligen Literaturstellen beschrieben.
Bevorzugt ist die Verwendung von Carbodiimiden, z.B. Dicyclohexylcarbodiimid oder Diisopropylcarbodiimid. Bevorzugt verwendet werden ebenfalls Phosphonium-Reagenzien, wie z.B. PyBOP oder PyBroP, Uronium-Reagenzien, wie z.B. HBTU, TBTU, TPTU, TSTU, TNTU, TOTU oder HATU, BOI oder Säurechloride bzw. Säurefluoride.

Die Kupplung kann dabei direkt durch Addition von Aminosäurederivat oder PNA-Monomer der Formel IV mit dem Aktivierungsreagenz und gegebenenfalls unter Zusatz von Additiven wie z.B. 1-Hydroxybenzotriazol (HOBt) (W. König, R. Geiger, Chem. Ber. 103, 788 (1970)) oder 3-Hydroxy-4-oxo-3,4-dihydrobenzotriazin (HOObt) (W. König, R. Geiger, Chem. Ber. 103, 2034 (1970)) zum Harz durchgeführt werden oder aber die Voraktivierung des Bausteines als aktivierter Ester kann separat erfolgen und die Lösung der aktivierten Spezies in einem geeigneten Lösungsmittel zum kupplungsfähigen Polymer gegeben werden.

Bei den in der exocyclischen Aminofunktion geschützten Nucleobasen B' werden für den Schutz der exocyclischen Aminofunktion mit der basenlabilen Aminoschutzgruppe PG kompatible Schutzgruppen wie z.B. gegen schwache oder mittelstarke Säuren labile Schutzgruppen vom Urethantyp wie tert. Butyloxycarbonyl (Boc), 4-Methoxybenzyloxycarbonyl (Moz), 3.5-Dimethoxyphenyl-2-propyl-2-oxycarbonyl (Ddz) oder vom Trityl-Typ wie Triphenylmethyl (Trt), (4-Methoxyphenyl)diphenylmethyl (Mmt), (4-Methylphenyl)diphenylmethyl (Mtt), Di-(4-methoxyphenyl)phenylmethyl (Dmt), 9-(9-Phenyl)xanthenyl (Pixyl) verwendet. Besonders bevorzugt ist die Verwendung von Butyloxycarbonyl (Boc), Triphenylmethyl (Trt), (4-Methoxyphenyl)diphenylmethyl (Mmt), (4-Methylphenyl)diphenylmethyl (Mtt), Di-(4-methoxyphenyl)phenylmethyl (Dmt) wobei Trt, Mtt, Mmt und Dmt überraschenderweise die Löslichkeit der Monomeren deutlich verbessern. Ganz besonders bevorzugt ist die Verwendung von (4-Methoxyphenyl)diphenylmethyl (Mmt).

Abspaltungsreagenzien für die basenlabile Aminoschutzgruppe PG sind beispielsweise eine Lösung von Piperidin, Morpholin, Hydrazin oder 1,8-Diazabicyclo[5.4.0]undec-7-en (DBU) in Dimethylformamid, N-Methylpyrrolidinon (NMP), Acetonitril (ACN) oder Dichlormethan (DCM), bevorzugt sind insbesondere für die Fmoc, Dnpeoc und Bnpeoc-Schutzgruppe eine Verwendung von 20% Piperidin in DMF oder N-Methylpyrrolidinon sowie einer Mischung von 2% DBU und 2% Piperidin in DMF oder 0.1M DBU in DMF oder 0.1M DBU in Dichlormethan.

Die Aufkupplung der Aminosäurereste Q' bzw. A' der Formel la erfolgt mit Aminosäurederivaten die vorzugsweise die gleiche Aminoschutzgruppe PG tragen, die auch für die Verbindungen der Formel IV verwendet werden. Eventuell vorhandene Seitenkettenfunktionen der Aminosäuren sind mit einer gegen schwache bis mittelstarke Säuren labilen Schutzgruppen, wie z.B. Boc, Moz, OtBu, tBu, Trt, Mtr, Pmc versehen. Bevorzugt sind hierbei Aminosäurederivate wie PG-Gly-OH, PG-Lys(Boc)-OH, PG-Arg(Mtr)-OH, PG-Arg(Pmc)-OH, PG-Arg(Trt)-OH, PG-Cys(Trt)-OH, PG-Asp(OtBu)-OH, PG-Glu(OtBu)-OH, PG-Aeg(Boc)-OH, PG-His(Trt)-OH, worin PG die obige Bedeutung hat. Ganz besonders bevorzugt sind hier die folgenden Aminosäurederivate: Fmoc-Gly-OH, Fmoc-Lys(Boc)-OH, Fmoc-Arg(Mtr)-OH, Fmoc-Arg(Pmc)-OH, Fmoc-Arg(Trt)-OH, Fmoc-Cys(Trt)-OH, Fmoc-Asp(OtBu)-OH, Fmoc-Glu(OtBu)-OH, Fmoc-Aeg(Boc)-OH, Fmoc-His(Trt)-OH.

Die bei dem oben beschriebenen Syntheseverfahren eingesetzten Verbindungen der Formel IV 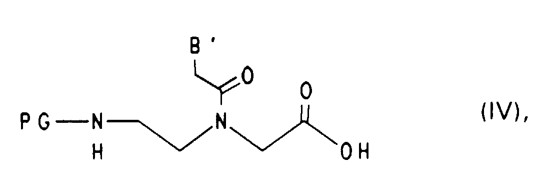 worin
- PG: für eine basenlabile Aminoschutzgruppe und
- B': für eine Nucleobase stehen, deren exocyclische Aminofunktion durch eine mit der basenlabilen Aminoschutzgruppe Kompatiblen Schutzgruppe geschützt ist, sind neu.

Die Verbindungen der Formel IV 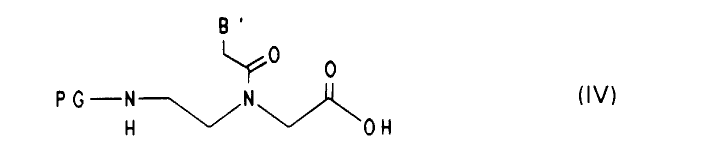 erhält man dadurch, daß man eine Verbindung der Formel V 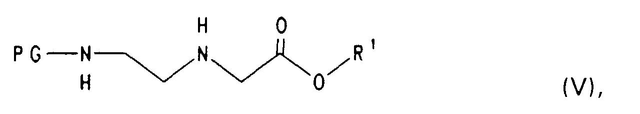 worin
- PG: eine basenlabile Aminoschutzgruppe und
- R¹: eine Esterschutzgruppe wie z.B. Methyl, Ethyl, Butyl, 2-(Methoxy-ethoxy)-ethyl, Benzyl, bevorzugt Methyl, 2-(Methoxy-ethoxy)-ethyl, besonders bevorzugt Methyl bedeuten
mit einer Verbindung der Formel VI 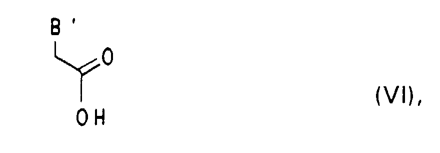 worin
- B': eine Nucleobase, wobei die exocyclische Aminofunktion der Nucleobase durch eine mit der basenlabilen Aminoschutzgruppe kompatiblen Schutzgruppe wie z.B. gegen schwache oder mittelstarke Säuren labile Schutzgruppen vom Urethantyp wie Boc, Moz oder vom Trityl-Typ wie Trt, Mmt, Dmt, Pixyl geschützt ist, bedeutet,
bei 0-45°C, bevorzugt bei Raumtemperatur, in einem geeigneten Lösungmittel, wie zum Beispiel DMF, Acetonitril, Dichlormethan oder Mischungen dieser Lösungsmittel unter Verwendung eines in der Peptidchemie üblichen Kupplungsreagenzes, wie z.B. Carbodiimiden, Phosphonium-Reagenzien, Uronium-Reagenzien, Säurehalogeniden, aktivierten Estern zu einer Verbindung der Formel VII 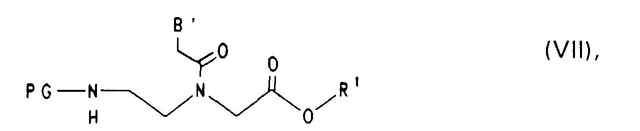 worin
- PG, B' und R¹: wie oben definiert sind,
umsetzt, und anschließend die Esterschutzgruppe R¹ mit Alkalilauge oder auch enzymatisch mit Hilfe von Esterasen oder Lipasen bei 0-50°C in einem geeigneten Lösungsmittel wie z.B. Dioxan, Wasser, Tetrahydrofuran, Methanol, Wasser oder Gemischen aus diesen Lösungsmitteln abspaltet.

Eine weitere Möglichkeit zur Synthese der Verbindung der der Formel IV besteht darin, daß man eine Verbindung der Formel V 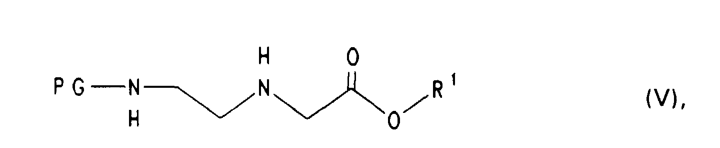 worin
- PG: eine basenlabile Aminoschutzgruppe in der obigen Bedeutung und
- R¹: Wasserstoff oder eine temporäre Silylschutzgruppe, wie z.B. Trimethylsilyl bedeuten,
mit einer Verbindung der Formel VIII

B' - CH₂- CO - R² (VIII),

worin
- B': wie in Formel VI definiert ist, und
- R²: Halogen, wie z.B. Fluor, Chlor, Brom oder den Rest eines Aktivesters, wie z.B. OBt, OObt, OPfp, ONSu, bedeuten
bei 0-40°C bevorzugt 20-30°C, in einem geeigneten Lösungsmittel, wie zum Beispiel DMF, NMP, Acetonitril, Dichlormethan oder Mischungen dieser Lösungsmittel umsetzt. Der temporäre Schutz der Säurefunktion in Verbindungen der Formel V kann dabei durch Umsetzung mit üblichen Silylierungsreagenzien wie z.B. Bis-Trimethylsilylacetamid erfolgen. Die Abspaltung dieser temporären Schutzgruppe erfolgt nach der Umsetzung mit Verbindungen der Formel VIII durch Zugabe von Wasser oder Alkoholen zur Reaktionsmischung.

Für die Synthese der Verbindungen der Formel V wird Aminoethylglycin oder der entsprechende Aminoethylglycinester mit der entsprechenden basenlabilen Schutzgruppe versehen. Die Einführung der basenlabilen Schutzgruppe erfolgt dabei mit literaturbekannten zum Teil modifizierten Verfahren. Geeignete Reagenzien sind z.B. Fmoc-Cl, Fmoc-ONSu, Bnpeoc-ONSu, Dnpeoc-ONSu, Msc-Cl, 2-Acetyldimedon (Dde). Bei dieser Umsetzung kann die Löslichkeit des Aminoethylglycins unter gleichzeitigem Schutz der Säurefunktion durch Umsetzung mit üblichen Silylierungsreagenzien wie z.B. Bis-Trimethylsilylacetamid verbessert werden. Die Abspaltung dieser temporären Schutzgruppe erfolgt nach der Umsetzung mit den Schutzgruppenreagenzien durch Zugabe von Wasser oder Alkoholen zur Reaktionsmischung. Das als Ausgangsmaterial verwendete Aminoethylglycin oder der entsprechende Aminoethylglycinester werden nach literaturbekannter Methode (E.P. Heimer, H.E. Gallo-Torres, A.M. Felix, M. Ahmad, T.J. Lambros, F. Scheidl and J. Meienhofer Int. J. Peptide Protein Res. 23, 1984, 203-211) hergestellt.

Ein weiteres einfacheres Verfahren zur Herstellung von Aminoethylglycin besteht in der reduktiven Aminierung von Glyoxylsäure mit Ethylendiamin und ist in der gleichzeitig eingereichten Anmeldung mit dem Titel "Verfahren zur Herstellung von Aminoethylglycin" (HOE 94/F 061, DE-P 44 08 530.3) beschrieben.

Die Nucleobasen-Essigsäurederivate der Formel VI werden durch Alkylierung der ensprechenden Nucleobasen oder in der exocyclischen Aminofunktion geschützten Nucleobasen mit Chloressigsäure, Bromessigsäure, Jodessigsäure oder deren Estern erhalten. Gegebenenfalls werden dabei zur selektiven Alkylierung zusätzlich temporäre Schutzgruppen an der Nucleobase eingeführt.

Als Schutzgruppe für die Nucleobasen können alle mit der basenlabilen Schutzgruppe PG kompatible Schutzgruppen verwendet werden. Für die exocyclische Aminofunktion werden bevorzugt die gegen schwache oder mittelstarke Säuren labilen Schutzgruppen vom Urethantyp wie Boc, Moz, Ddz oder vom Trityl-Typ wie Trt, Mmt, Dmt, Pixyl verwendet. Insbesondere bevorzugt sind die gegen schwache Säuren labilen Schutzgruppen vom Trityl-Typ wie Trt, Mmt, Dmt, besonders bevorzugt Mmt, wobei die letzteren überraschenderweise die Löslichkeit der monomeren Bausteine deutlich verbessern, so daß Lösungen für die Verwendung in automatischen Synthesegeräten, wie z.B. multiplen Peptidsynthesizern, hergestellt werden können.

Die bei der Herstellung der Verbindungen der Formel V verwendeten in der exocyclischen Aminofunktion geschützten Nucleobasen B' werden nach an sich literaturbekannten Verfahren aus den entsprechenden Nucleobasen unter Verwendung eines geeigneten Schutzgruppenreagenzes wie z.B. Boc₂O, Mozazid, Trt-Cl, Mtt-Cl, Mmt-CI, Dmt-CI, Pixyl-Cl hergestellt. Gegebenenfalls werden dabei zur selektiven Alkylierung zusätzlich temporäre Schutzgruppen an der Nucleobase eingeführt.

Die Aufkupplung der Nucleobasen-essigsäuren der Formel VI auf die mit der basenlabilen Schutzgruppe geschützten Aminoethylglycinderivate der Formel V erfolgt unter Verwendung von in der Peptidsynthese üblichen Aktivierungsmethoden wie sie oben beschrieben sind. Bevorzugt ist die Verwendung von Carbodiimiden, z.B. Dicyclohexylcarbodiimid oder Diisopropylcarbodiimid. Bevorzugt verwendet werden ebenfalls Phosphonium-Reagenzien, wie z.B. BOP (B. Castro, J.R. Dormoy, G. Evin and C. Selve, Tetrahedron Lett. 1975, 1219-1222) und Uronium-Reagenzien, wie z.B. HBTU (V. Dourtoglou, B. Gross, V. Lambropoulou, C. Zioudrou, Synthesis 1984, 572-574); TBTU, TPTU, TSTU, TNTU (R. Knorr, A. Trzeciak, W. Bannwarth and D. Gillessen, Tetrahedron Letters 1989, 1927-1930); TOTU (EP-A-0 460 446) oder Säurechloride bzw. Säurefluoride (L. A. Carpino, H. G. Chao, M. Beyermann and M. Bienert, J. Org. Chem., 56(1991), 2635; J.-N. Bertho, A. Loffet, C. Pinel, F. Reuther and G. Sennyey in E. Giralt and D. Andreu (Eds.) Peptides 1990, Escom Science Publishers B. V. 1991, pp. 53-54)

Die oben beschriebenen PNA's werden durch Festphasensynthese an einem geeigneten Trägermaterial (z.B. Polystyrol, mit Polyoxyethylen modifiziertem Polystyrol, wie z.B. ®Tentagel, ®Controlled Pore Glass) das mit einer Ankergruppe L versehen ist, die latent den Rest Q^{o} enthält, aufgebaut. Die Festphasensynthese beginnt am C-terminalen Ende des PNA mit der Kupplung eines mit einer basenlabilen Schutzgruppe geschützten Monomers oder Aminosäure, die gegebenenfalls in der Seitenkettenfunktion geschützt ist, an ein entsprechendes Harz.

Nach Abspaltung der basenlabilen Schutzgruppe des an das Harz gekuppelten Bausteines mit einem geeigneten Reagenz, wie oben beschrieben, werden die nachfolgenden geschützten Bausteine (PNA-Monomere und Aminosäurederivate) nacheinander in der gewünschten Reihenfolge aufgekuppelt. Die intermediär entstehenden, mit einer basenlabilen Schutzgruppe am N-Terminus geschützten PNA-Harze werden vor der Verknüpfung mit dem nachfolgenden PNA-Monomeren durch die vorbeschriebenen Reagenzien entblockiert.

Die Kupplung bzw. Aktivierung der Aminosäurederivate mit einem der oben genannten Aktivierungsreagenzien kann in Dimethylformamid, N-Methylpyrrolidinon, Acetonitril oder Methylenchlorid oder einer Mischung aus den genannten Lösungsmitteln durchgeführt werden. Das aktivierte Derivat wird üblicherweise in einem 1,5 bis 10 fachen Überschuß eingesetzt. In Fällen, in denen eine unvollständige Kupplung eintritt, wird die Kupplungsreaktion wiederholt, ohne Entblockierung der Aminogruppe des gerade aufgekuppelten Bausteins durchzuführen.

Verfahren zur Einführung des Restes R^{o} sind zum Beispiel für den Fall, daß dieser Rest eine Carbonsäurefunktion enthält, die oben für die Aufkupplung der Aminosäuren und PNA-Monomeren beschriebenen Methoden. Weitere Verfahren sind die Umsetzung von Isocyanaten wie z.B. Phenylisocyanat, Isothiocyanaten wie z.B. Fluoresceinisothiocyanat, Chlorameisensäurederivaten, wie z.B. Chlorformylcarbazol, Kohlensäureaktivestern wie z.B. Cholesterin-(4-nitrophenyl)carbonat, Acridinium-succinimidylcarbonat, Sulfochloriden wie z.B. Dansylchlorid etc.

Der vorhergehend beschriebene Syntheseablauf kann auch mittels kommerziell erhältlicher Syntheseautomaten, wie z.B. Peptidsynthesizern, Multiplen Peptidsynthesizern, DNA-Synthesizern unter leichter Modifikation der üblicherweise verwendeten Syntheseprogramme durchgeführt werden.

Nach Synthese der PNA's in der vorgehend beschriebenen Weise kann das PNA-Oligomer vom Harz mit geigneten Reagenzien, wie z.B. Trifluoressigsäure bei gegen schwache oder mittelstarke Säuren labilen Ankergruppen abgespalten werden. Je nach Art des verwendeten Linkers und der Art der verwendeten Schutzgruppen werden Oligomer und die weiteren Seitenkettenschutzgruppen der Nucleinbasen simultan abgespalten. Das Abspaltungsreagenz kann dabei auch durch geeignete Lösungsmittel, wie z.B. Methylenchlorid, verdünnt angewendet werden. Gegebenenfalls können zur Vermeidung von Nebenreaktionen weitere Additive diesem Abspaltungsreagenz zugefügt werden. Als Kationenfänger eignen sich hierbei Substanzen wie Phenol, Kresol, Thiokresol, Anisol, Thioanisol, Ethandithiol, Dimethylsulfid, Ethylmethylsulfid, Triethylsilan, Triisopropylsilan oder ähnliche in der Festphasenpeptidsynthese übliche Additive, die einzeln oder als Mischung von zwei oder mehr dieser Hilfsmittel zugesetzt werden können.

Die Reinigung des nach der Abspaltung erhaltenen Roh-Oligomeres erfolgt mit in der Peptid- oder Nucleotidchemie üblichen Verfahren, wie z.B. HPLC, Ionenaustauschchromatographie etc.

Die für Aminosäuren verwendeten Abkürzungen entsprechen dem in der Peptidchemie üblichen drei Buchstaben-Code wie er in Europ. J. Biochem. 138, 9 (1984) beschrieben ist. Weitere verwendete Abkürzungen sind nachfolgend aufgelistet.
- ACN: Acetonitril
- Aeg: N-(2-Aminoethyl)glycyl, -NH-CH₂-CH₂-NH-CH₂-CO-
- Aeg(A^{Mmt}): N-(2-Aminoethyl)-N-((9-(N⁶-4-methoxyphenyl-diphenyl)adenosyl)acetyl)-glycyl
- Aeg(C^{Mmt}): N-(2-Aminoethyl)-N-((1-(N⁴-4-methoxyphenyl-diphenyl)cytosyl)acetyl)-glycyl
- Aeg(T): N-(2-Aminoethyl)-N-((1-thyminyl)acetyl)-glycyl
- Aeg(T^{Bom}): N-(2-Aminoethyl)-N-((1-(N³-benzyloxymethyl)-thyminyl)-acetyl)glycyl
- Aeg(T^{triazolo}): N-(2-Aminoethyl)-N-((2-hydroxy-5-methyl-4-triazolopyrimidin-1-yl)acetyl)-glycyl
- Bnpeoc: 2,2-[Bis(4-nitrophenyl)]-ethoxycarbonyl)
- Boc: tert.-Butyloxycarbony
- BOI: 2-(Benzotriazol-1-yl)oxy-1,3-dimethyl-imidazolidiniumhexafluorphosphat
- Bom: Benzyloxymethyl
- BOP: Benzotriazolyl-1-oxy-tris(dimethylamino)-phosphoniumhexafluorphosphat
- BroP: Brom-tris(dimethylamino)-phosphonium-hexafluorphosphat
- BSA: N,O-Bis-(trimethylsilyl)-acetamid
- But: tert.-Butyl
- Bzl: Benzyl
- Cl-Z: 4-Chlor-benzyloxycarbonyl
- CPG: Controlled Pore Glas
- DBU: 1,8-Diazabicyclo[5.4.0]undec-7-en
- DCM: Dichlormethan
- Dde: 1-(4,4-Dimethyl-2,6-dioxocyclohexyliden)ethyl
- Ddz: 3.5-Dimethoxyphenyl-2-propyl-2-oxycarbonyl
- DMF: Dimethylformamid
- DMT: 4,4'-Dimethoxytriphenylmethyl
- Dmt: Di-(4-methoxyphenyl)phenylmethyl,
- Dnpeoc: 2-(2,4-Dinitrophenyl)-ethoxycarbonyl
- FAM: Fluorescein-Rest
- Fmoc: 9-Fluorenylmethyloxycarbonyl
- H-Aeg-OH: N-(2-Aminoethyl)glycin
- HAPyU: O-(7-Azabenzotriazol-1-yl)-1,1,3,3-bis(tetramethylen)uroniumhexafluorphosphat
- HATU: O-(7-Azabenzotriazol-1-yl)-1,1,3,3-tetramethyluroniumhexafluorphosphat
- HBTU: O-(Benzotriazol-1-yl)-1,1,3,3-tetramethyluronium-hexafluorphosphat
- HOBt: 1-Hydroxybenzotriazol
- HONSu: N-Hydroxysuccinimid
- HOObt: 3-Hydroxy-4-oxo-3,4- dihydrobenzotriazin
- MeOBz: 4-Methoxybenzoyl
- Mmt: 4-Methoxytriphenylmethyl
- Moz: 4-Methoxybenzyloxycarbonyl
- MSNT: 2,4,6-Mesitylensulfonyl-3-nitro-1,2,4-triazolid
- Mtt: 4-Methylphenyl)diphenylmethyl
- NBA: Nitrobenzylalkohol
- NMP: N-Methylpyrrolidin
- Pixyl: 9-(9-Phenyl)xanthenyl
- PyBOP: Benzotriazolyl-1-oxy-tripyrrolidinophosphonium-hexafluorphosphat
- PyBroP: Brom-tripyrrolidinophosphonium-hexafluorphosphat
- TAPipU: O-(7-Azabenzotriazol-1-yl)-1,1,3,3-bis(pentamethylen)uroniumtetrafluoroborat
- TBTU: O-(Benzotriazol-1-yl)-1,1,3,3-tetramethyluronium-tetrafluoroborat
- tBu: tert.-Butyl
- tBuBz: 4-tert-Butylbenzoyl
- TDBTU: O-(3,4-Dihydro-4-oxo-1,2,3-benzotriazin-3-yl)-1,1,3,3-tetramethyluronium-tetrafluoroborat
- TDO: 2,5-Diphenyl-2,3-dihydro-3-oxo-4-hydroxythiophendioxid
- TFA: Trifluoressigsäure
- THF: Tetrahydrofuran
- TNTU: O-[(5-Norbornen-2,3-dicarboximido]-1,1,3,3-tetramethyluronium-tetrafluoroborat
- TOTU: O-[(Cyano(ethoxycarbonyl)methylen)amino]-1,1,3,3-tetramethyluronium-tetrafluoroborat
- TPTU: O-(1,2-Dihydro-2-oxo-1-pyridyl)-1,1,3,3'-tetramethyluroniumtetrafluoroborat
- Trt: Trityl
- TSTU: O-(N-Succinimidyl)-1,1,3,3-tetramethyluronium-tetrafluoroborat
- Z: Benzyloxycarbonyl
- MS(ES⁺): Elektrospray Massenspektrum (Positiv lon)
- MS(ES⁻): Elektrospray Massenspektrum (Negativ lon)
- MS(DCI): Desorption Chemical Ionisation Massenspektrum
- MS(FAB): East-Atom-Bombardment-Massenspektrum

Die folgenden Beispiele sollen die bevorzugten Methoden zur Herstellung der erfindungsgemäßen Verbindungen verdeutlichen, ohne daß die Erfindung darauf eingeschränkt wäre.

### Beispiel 1

### N-(Fmoc-aminoethyl)glycinmethylester-Hydrochlorid

### Fmoc-Aeg-OMe · HCl

8.2 g Aminoethylglycinmethylester-Dihydrochlorid werden in 100 ml Wasser gelöst und unter kräftigem Rühren 13.48 g Fmoc-ONSu in 400 ml Dioxan zugegeben. Dann tropft man 10.08 g Natriumhydrogencarbonat in 150 ml Wasser innerhalb 15 min zu. Man läßt noch 15 Std. bei Raumtemperatur rühren und engt dann im Vakuum auf ca. 100 ml ein. Man extrahiert mit 600 ml Ethylacetat und wäscht die organische Phase mit 3 mal 50 ml Wasser. Die organische Phase wird über Natriumsulfat getrocknet und dann auf ca. 50 ml Volumen eingeengt. Man versetzt mit 100 ml Dichlormethan und 20 ml 2N methanolischer Salzsäure wobei das Produkt sofort ausfällt. Man läßt noch über Nacht im geschlossenen Kolben bei 4°C stehen. Der Niederschlag wird abgesaugt und getrocknet.
Ausbeute: 9.8 g
R_{F}: 0.50 (n-Butanol/Essigsäure/Wasser 3:1:1)
MS(FAB, NBA/LiCl): 361.2 [M+Li]⁺

### Beispiel 2

### N-(Fmoc-aminoethyl)glycin-Hydrochlorid

### Fmoc-Aeg-OH · HCl

11.21 g Aminoethylglycin werden in 250 ml DMF suspendiert und unter Rühren mit 98.8 ml Bis(trimethylsilyl)acetamid versetzt. Nach 15 min Rühren erhält man eine klare Lösung. Dazu tropft man innerhalb 15 min eine Lösung von 33.7 g Fmoc-ONSu in 200 ml DMF. Man rührt noch 1 Std. nach und gibt dann je 100 ml Methanol und Wasser zur Reaktionsmischung. Nach 10 min werden noch 16.6 ml 6N Salzsäure zugegeben und weitere 10 min gerührt. Danach wird die Mischung am Rotationsverdampfer im Vakuum zur Trockene eingeengt und der Rückstand mit 250 ml Dichlormethan versetzt und gerührt. Zuerst erhält man eine klare Lösung aus der dann nach ca. 5 min das Produkt langsam ausfällt. Nach und nach werden weitere 250 ml Dichlormethan zugegeben und insgesamt 1.5 Std gerührt. Das ausgefallene Produkt wird scharf abgesaugt und dann auf der Nutsche mit zweimal je 150 ml Dichlormethan verrührt und abgesaugt. Der Niederschlag wird anschließend in einen Kolben überführt und mit ca. 300 ml Ethylacetat bei 45°C ausgerührt, warm abfiltriert und mit wenig warmen Ethylacetat nachgewaschen. Der Niederschlag wird im Vakuumexsiccator gut getrocknet.
Ausbeute: 34.4 g

### Beispiel 3

### Freisetzung von Fmoc-Aminoethylglycin

### Fmoc-Aeg-OH

23 g des obigen Fmoc-Aminoethylglycin-Hydrochlorids werden in 400 ml Methanol heiß gelöst. Nach dem Abkühlen auf Raumtemperatur wird unter gutem Rühren eine Lösung von 2.2 g NaOH in 80 ml Methanol zugetropft. Dabei fällt das Produkt sofort aus. Man gibt noch 40 ml Wasser zur Lösung und erhitzt diese Mischung, wobei eine klare Lösung entsteht, die man langsam auf Raumtemperatur abkühlen und dann noch 1 Std bei 0°C stehen läßt. Der Niederschlag wird abgesaugt, dreimal mit je 70 ml 90%-igem wäßrigen Methanol gewaschen und dann im Vakuum-Exsiccator gut getrocknet.
Ausbeute: 15.0 g
R_{F}: 0.55 (n-Butanol/Essigsäure/Wasser 2:1:2)
MS(FAB, NBA): 341.2 [M+H]⁺

### Beispiel 4

### 2-Amino-6-chlor-purin-9-yl-essigsäuremethylester

In einer gut getrockneten Glasapparatur werden unter Argonatmosphäre 1.43 g Natriumhydrid (95%) vorgelegt und mit 200 ml gut getrocknetem N, N-Dimethylformamid übergossen. Dann werden 10.0 g 2-Amino-6-chlorpurin zugegeben wobei Gasentwicklung und Erwärmung auftritt und eine gelbe Lösung entsteht. Nach einer Stunde wird unter gutem Rühren eine Lösung von 10.8 g Bromessigsäuremethylester in 40 ml DMF zugetropft. Man läßt noch 2 h nachrühren und engt die Reaktionsmischung dann am Rotationsverdampfer im Ölpumpenvakuum ein. Der Rückstand wird mit Wasser versetzt und dann mit Ethylacetat extrahiert. Die organische Phase wird zur Trockene eingeengt und das Rohprodukt mittels Flash-Chromatographie an Kieselgel mit Dichlormethan/ Methanol (95/5) gereinigt.
Ausbeute: 11 g Produkt
R_{F}: 0.44 (Dichlormethan/Methanol 9:1)
MS(CI): 242(M+H)⁺

### Beispiel 5

### 2-(4-Methoxyphenyl-diphenylmethyl-amino)-6-chlor-purin-9-yl-essigsäuremethylester

11 g 2-Amino-6-chlor-purin-9-yl-essigsäuremethylester werden in einer Mischung aus 75 ml Pyridin und 9.5 ml Triethylamin unter Argon suspendiert und dann 15 g 4-Methoxyphenyl-diphenylmethylchlorid in 4 Portionen innerhalb einer Stunde zugegeben. Man läßt über Nacht nachreagieren und engt dann das Gemisch zur Trockene ein, koevaporiert einmal mit wenig Toluol und reinigt das Rohprodukt mittels Flash-Chromatographie an Kieselgel mit Dichlormethan/Methanol (98:2).
Ausbeute: 15.5 g
R_{F}: 0.60 (Dichlormethan/Methanol 97:3)
MS(ES⁺): 514.2(M+H)⁺

### Beispiel 6

### 2-(4-Methoxyphenyl-diphenylmethyl-amino)-6-hydroxy-purin-9-yl-essigsäure

0.5 g 2-(4-Methoxyphenyl-diphenylmethyl-amino)-6-chlor-purin-9-yl-essigsäuremethylester werden 3 h in 12 ml 10%iger Natronlauge unter Rückfluß erhitzt. Dann läßt man auf Raumtemperatur abkühlen und neutralisiert vorsichtig mit 10%iger HCI. Das ausgefallene Produkt wird abgesaugt, mehrfach mit Wasser gewaschen und gut getrocknet.
Ausbeute: 400 mg
R_{F}: 0.1 (Dichlormethan/Methanol 7:3)
MS(FAB, NBA/LiCl): 488.2(M+Li)⁺

### Beispiel 7

### 6-(4-Methoxyphenyl-diphenylmethyl-amino)-purin

13.5 g 6-Aminopurin und 46.2 g 4-Methoxyphenyl-diphenylmethylchlorid werden in 500 ml trockenem Dimethylformamid suspendiert und nach Zugabe von 13.9 ml Triethylamin kurz auf 60°C erhitzt. Die fast klare, grünliche Lösung wird über Nacht stehengelassen. Dann wird die Mischung am Rotationsverdampfer im Vakuum eingeengt und nach Versetzen mit 50 ml Methanol nochmal eingeengt. Der Rückstand wird an Kieselgel mit Dichlormethan/Methanol (95:5) unter Zusatz von 0.1% Triethylamin chromatographisch gereinigt.
Ausbeute: 9.8 g eines Schaumes
R_{F}: 0.35 (Dichlormethan/Methanol 9:1)
MS(FAB,MeOH/NBA): 408.2(M+H)⁺

### Beispiel 8

### 6-(4-Methoxyphenyl-diphenylmethyl-amino)-purin

20.25 g 6-Aminopurin und 69.3 g 4-Methoxyphenyl-diphenylmethylchlorid werden in 500 ml trockenem Pyridin suspendiert und nach Zugabe von 19.1 ml 4-Ethylmorpholin kurz auf ca. 40°C erhitzt. Die Mischung wird über Nacht stehengelassen. Dann wird die Suspension mit Wasser und Dichlormethan verrührt und der Niederschlag abgesaugt. Die Dichlormethan-Phase wird im Vakuum zur Trockene eingengt, mit wenig Dichlormethan verrieben, der Niederschlag abgesaugt und mit dem zuerst erhaltenen vereinigt. Nach Trocknung erhält man 47.3 g Produkt.
R_{F}: 0.53 (Ethylacetat)
MS(FAB, MeOH/NBA): 408.2(M+H)⁺

### Beispiel 9

### 6-(4-Methoxyphenyl-diphenylmethyl-amino)-purin-9-yl-essigsäuremethylester

6.2 g 6-(4-Methoxyphenyl-diphenylmethyl-amino)-purin werden in 75 ml trockenem Dimethylformamid verteilt und dann 0.36 g Natriumhydrid zugegeben. Man rührt noch eine Stunde nach und gibt dann 2.52 g Bromessigsäuremethylester hinzu und rührt weitere 2h. Dann wird die Reaktionsmischung mit 2 ml Methanol versetzt, 10 min nachgerührt und dann im Vakuum am Rotationsverdampfer eingeengt. Der Rückstand wird an Kieselgel mit Ethylacetat unter Zusatz von 0.5% Triethylamin chromatographisch gereinigt.
Ausbeute: 4.0 g eines Schaumes
R_{F}: 0.68 (Ethylacetat/Methanol 3:1)
MS(FAB, NBA/LiCl): 480.2(M+H)⁺; 485.2(M+Li)⁺

### Beispiel 10

### 6-(4-Methoxyphenyl-diphenylmethyl-amino)-purin-9-yl-essigsäure

3.0 g 6-(4-Methoxyphenyl-diphenylmethyl-amino)-purin-9-yl-essigsäuremethylester werden in 20 ml Dimethylformamid gelöst und unter Rühren mit 0.25 g NaOH in 5 ml Wasser versetzt. Nach 10 min ist die Verseifung beendet und das Reaktionsgemisch wird mit verdünnter Essigsäure auf pH 6 gestellt. Man verdünnt noch mit 100 ml Wasser und engt dann im Vakuum am Rotationsverdampfer ein. Anschließend wird noch zweimal mit wenig Toluol nachdestilliert und der Rückstand mit Diethylether versetzt wobei das Produkt auskristallisiert. Man saugt das ausgefallene Produkt ab, wäscht mit wenig Diethylether nach und trocknet.
Ausbeute: 3.2 g
Schmelzpunkt: 157-160°C(Zers.)
R_{F}: 0.15 (Ethylacetat/Methanol 3:1)
MS(FAB, NBA/LiCl): 465.1(M+H)⁺; 472.1(M+Li)⁺; 478.2(M+2Li)⁺

### Beispiel 11

### 1-Acetyl-2.4-dihydroxy-5-methyl-pyrimidin

### 1-Acetyl-thymin

75 g Thymin werden in 375 ml Acetanhydrid suspendiert und 45 min unter Rückfluß erhitzt. Danach wird die Mischung auf 0°C gekühlt wobei das Produkt ausfällt. Der Niederschlag wird abgesaugt und mit 375 ml Ethylacetat verrührt. Man saugt den Niederschlag ab, wäscht mit etwas Diethylether nach und trocknet.
Ausbeute: 89.1 g
Schmelzpunkt: 187-189°C
R_{F}: 0.67 (Dichlormethan/Methanol 95:5)

### Beispiel 12

### 3-Benzyloxymethyl-2.4-dihydroxy-5-methyl-pyrimidin

### 3-Benzyloxymethyl-thymin

52.0 g 1-Acetyl-2.4-dihydroxy-5-methyl-pyrimidin werden in 300 ml DMF suspendiert und nach Zugabe von 47.5 ml Triethylamin auf 0°C gekühlt. Zur gut gerührten Mischung werden langsam 100 ml Benzylchlormethylether in 50 ml DMF bei dieser Temperatur zugetropft. Man rührt noch 1 h nach und läßt über Nacht bei Raumtemperatur stehen. Dann wird die Reaktionsmischung im Vakuum am Rotationsverdampfer eingeengt, in 450 ml Methanol und 300 ml einer 40%igen wäßrigen Lösung von Methylamin aufgenommen und 1.5 h am Rückfluß gekocht. Die Lösung wird dann eingeengt und mit 10.8 g Natriumhydrogencarbonat in 600 ml Wasser verrührt. Das ausgefallene Produkt wird abgesaugt, mit Wasser und Ethanol gewaschen und aus Ethanol umkristallisiert.
Ausbeute: 46.66 g
Schmelzpunkt: 119-120°C
R_{F}: 0.38 (Dichlormethan/Methanol 95:5)

### Beispiel 13

### 3-Benzyloxymethyl-2.4-dihydroxy-5-methyl-pyrimidin-essigsäureethylester

### 3-Benzyloxymethyl-thyminyl-essigsäureethylester

4.97g Natriumhydrid werden in 150 ml THF vorgelegt, dann 45.04 g 3-Benzyloxymethyl-2.4-dihydroxy-5-methyl-pyrimidin gelöst in 550 ml THF unter N₂-Atmosphäre bei 0°C zugetropft und anschließend 21,42 ml Bromessigsäureethylester in 700 ml THF bei dieser Temperatur zugegeben. Danach läßt man noch 5 h bei Raumtemperatur nachrühren und gibt anschließend vorsichtig Wasser hinzu. Die organische Phase wird abgetrennt und die Wasserphase noch viermal mit Dichlormethan extrahiert. Die vereinigten organischen Phasen werden über Magnesiumsulfat getrocknet, eingeengt und der Rückstand mit Heptan verrührt.
Ausbeute: 52.4 g
R_{F}: 0.15 (Dichlormethan/Methanol 95:5)
MS(FAB, NBA): 319.1(M+H)⁺

### Beispiel 14

### 2.4-Dihydroxy-5-methyl-pyrimidin-essigsäureethylester

### Thyminyl-essigsäureethylester

25 g 3-Benzyloxymethyl-2.4-dihydroxy-5-methyl-pyrimidin-essigsäureethylester werden in 1000 ml Dichlormethan gelöst, die Mischung auf -70°C abgekühlt und bei dieser Temperatur unter gutem Rühren 375 ml einer 1M Lösung von Bortrichlorid in Dichlormethan zugetropft. Man läßt noch 3 h bei dieser Temperatur nachreagieren und tropft dann bei -70° bis -60°C 320 ml Methanol zu. Nach 1 h gibt man 197.05 ml Triethylamin hinzu und läßt die Mischung auf Raumtemperatur kommen. Dann gibt man noch etwas Wasser hinzu und engt das Gemisch am Rotationsverdampfer ein. Der Rückstand wird in Ethylacetat aufgenommen und mit Wasser gewaschen. Die organische Phase wird über Magnesiumsulfat getrocknet und dann zur Trockene eingeengt.
Ausbeute: 12 g
R_{F}: 0.45 (Dichlormethan/Methanol 9:1)
MS(DCI): 199(M+H)⁺

### Beispiel 15

### 2.4-Dihydroxy-5-methyl-pyrimidin-essigsäure

### Thyminyl-essigsäure

10 g 2.4-Dihydroxy-5-methyl-pyrimidin-essigsäureethylester werden in 130 ml Dioxan/Wasser (1:1) gelöst und dann nach Zugabe von 60 ml 1N LiOH über Nacht bei Raumtemperatur gerührt. Dann wird die Mischung auf kleines Volumen eingeengt, die wäßrige Phase mit Ether gewaschen und dann auf pH 2.5 gestellt. Hierbei fällt das Produkt aus. Man saugt ab und erhält 4.9 g Produkt. Aus der Mutterlauge lassen sich durch extrahieren mit Pentanol und ausfällen mit Heptan/Ether weitere 1.5 g Produkt erhalten.
Ausbeute: 6.4 g
R_{F}: 0.30 (Dichlormethan/Methanol 3:2)
MS(DCI): 185(M+H)⁺

### Beispiel 16

### N-1 Chlorocarboxymethylthymin

### Thyminyl-essigsäurechlorid

### Beispiel 16a

2 g 2.4-Dihydroxy-5-methyl-pyrimidin-essigsäure werden 3h mit 15 ml Thionylchlorid bei 60°C gerührt bis keine Gasentwicklung mehr auftritt. Dann wird das überschüssige Thionylchlorid am Rotationsverdampfer im Vakuum abgezogen und noch dreimal mit wenig Toluol nachdestilliert. Das so erhaltene Produkt wird direkt in die nachfolgende Reaktion eingesetzt.
Ausbeute: 2.4 g
MS(DCI): 203(M+H)⁺

### Beispiel 16b

N-1 Carboxymethylthymin (3.0 g; 16.3 mmol) werden in Thionylchlorid (90 ml) suspendiert. Die Suspension wird für 1.5h auf 70°C erhitzt und dann 16 h bei Raumtemperatur stehengelassen. Das überschüssige Thionylchlorid wird in Vakuum entfernt und der Rückstand dreimal mit Toluol koevaporiert. Das erhaltene Produkt wird zweimal mit n-Hexan verrieben und im Vakuum getrocknet. Man erhält die Verbindung als hell-oranges Pulver.
Ausbeute: 3.30 g
MS(Cl; Dichlormethan): 203 (M+H)⁺
R_{f} = 0.10 (Dichlormethan/Methanol 7:3).

### Beispiel 17

### 2-Hydroxy-4-(4-Methoxyphenyl-diphenylmethyl-amino)-pyrimidin

### N⁴-Mmt-cytosin

11.6 g 4-Amino-2-hydroxypyrimidin und 46.2 g 4-Methoxyphenyl-diphenylmethylchlorid werden in 500 ml trockenem Pyridin suspendiert und nach Zugabe von 12.8 ml 4-Ethylmorpholin kurz auf ca. 40°C erhitzt. Die Mischung wird über Nacht stehengelassen. Dann wird die Suspension mit Wasser und Dichlormethan verrührt und der Niederschlag abgesaugt. Die Dichlormethan-Phase wird im Vakuum zur Trockene eingengt, mit wenig Dichlormethan verrieben, der Niederschlag abgesaugt und mit dem zuerst erhaltenen vereinigt. Nach Trocknung erhält man 16.8 g Produkt.
R_{F}: 0.45 (Dichlormethan/Methanol 9:1)
MS(FAB,MeOH/NBA): 384.2(M+H)⁺

### Beispiel 18

### 2-Hydroxy-4-(4-Methoxyphenyl-diphenylmethyl-amino)-pyrimidin-1-yl-essigsäure-methylester

### N⁴-Mmt-cytosyl-essigsäuremethylester

11.5 g 2-Hydroxy-4-(4-Methoxyphenyl-diphenylmethyl-amino)-pyrimidin werden in 120 ml trockenem Dimethylformamid verteilt und dann 0.72 g Natriumhydrid zugegeben. Man rührt noch eine Stunde nach und gibt dann 5.05 g Bromessigsäuremethylester hinzu und rührt weitere 2 h. Dann wird die Reaktionsmischung mit 2 ml Methanol versetzt, 10 min nachgerührt und dann im Vakuum am Rotationsverdampfer eingeengt.Der Rückstand wird Wasser verrieben, abgesaugt und getrocknet.
Ausbeute: 10.7 g
R_{F}: 0.39 (Ethylacetat)
MS(FAB, NBA/LiCI): 462.2(M+Li)⁺

### Beispiel 19

### 2-Hydroxy-4-(4-Methoxyphenyl-diphenylmethyl-amino)-pyrimidin-1-yl-essigsäure

### N⁴-Mmt-cytosyl-essigsäure

10.5 g 2-Hydroxy-4-(4-Methoxyphenyl-diphenylmethyl-amino)-pyrimidin-1-yl-essigsäure-methylester werden in 100 ml Dimethylformamid gelöst und unter Rühren mit 0.94 g NaOH in 5 ml Wasser versetzt. Nach 10 min ist die Verseifung beendet und das Reaktionsgemisch wird mit verdünnter Essigsäure auf pH 6 gestellt. Man verdünnt noch mit 100 ml Wasser und engt dann im Vakuum am Rotationsverdampfer ein. Anschließend wird noch zweimal mit wenig Toluol nachdestilliert und der Rückstand mit Diethylether versetzt wobei das Produkt ausfällt. Man saugt das ausgefallene Produkt ab, wäscht mit wenig Diethylether nach und trocknet.
Ausbeute: 8.55 g
R_{F}: 0.27 (Ethylacetat)
MS(FAB, NBA/LiCl): 448 (M+Li)⁺

### Beispiel 20

### N⁴-(tert.Butyloxycarbonyl)-N¹-carboxymethyl cytosin

### N⁴-Boc-cytosyl-essigsäure

### Beispiel 20a

### N⁴-(tert.Butyloxycarbonyl)-cytosin

### N⁴-Boc-cytosin

Cytosin (11.1 g) wird in trockenem Pyridin (250 ml) suspendiert. Dann werden Di-tert.butyldicarbonat (21.8 g) und eine Spatelspitze 4-Dimethylaminopyridin zugegeben. Die Mischung wird 6 h bei 60°C gerührt wobei ein dicker Niederschlag entsteht. Die Reaktionslösung wird abgekühlt und der Niederschlag abgesaugt. Der Niederschlag wird in der Wärme mit Wasser verrührt, abgesaugt und im Vakuum getrocknet.
Ausbeute: 10.26g
MS(DCI): 212 (M+H)⁺.
R_{F}: 0.6 (Dichlormethan/Methanol 6:4)

### Beispiel 20b

### N⁴-(tert.Butyloxycarbonyl)-N¹-methoxycarbonylmethyl cytosin

### N⁴-Boc-cytosyl-essigsäuremethylester

N⁴-(tert.Butyloxycarbonyl)-cytosin (1.6 g) werden in trockenem DMF (30 ml) suspendiert, Natriumhydrid (0.19 g) in Portionen zugegeben und die Mischung 1.5 h bei Raumtemperatur gerührt bis die Wasserstoffentwicklung beendet ist. Bei Raumtemperatur wird anschließend mit einer Spritze Bromessigsäuremethylester (0.84 ml) tropfenweise zugegeben. Man rührt noch 5 h bei Raumtemperatur nach und versetzt dann mit Methanol (1 ml). Das Lösungsmittel wird im Vakuum abgezogen und der verbliebene Rückstand mittels Säulenchromatographie an Kieselgel mit Dichlormethan als Elutionsmittel gereinigt. Die das Produkt enthaltenden Fraktionen werden vereinigt und im Vakuum eingeengt.
Ausbeute 1.1g
MS(DCI): 284 (M+H)⁺.
R_{F}: 0.5 (Dichlormethan/Methanol 95:5)

### Beispiel 20c

### N⁴-(tert.Butyloxycarbonyl)-N¹-carboxymethyl cytosin

### N⁴-Boc-cytosyl-essigsäure

N⁴-(tert.Butyloxycarbonyl)-N¹-methoxycarbonylmethyl cytosin (4.2 g) wird in Wasser (30 ml) suspendiert und bei 0°C tropfenweise mit 2N wäßriger Natronlauge unter pH-Kontrolle (pH 12) versetzt bis der Methylester verseift ist. Der Fortgang der Reaktion wird mittels DC verfolgt. Dann wird die Reaktionslösung mit Essigsäure auf pH 3 gestellt und das Lösungsmittel im Vakuum abdestilliert. Die Reinigung des Rohproduktes erfolgt mit Säulenchromatographie an Kieselgel mit Dichlormethan/Methanol/Triethylamin 8:1:1 als Elutionsmittel. Die das Produkt enthaltenden Fraktionen werden vereinigt und im Vakuum eingeengt.
Ausbeute: 3.5 g
Schmelzpunkt: 95-98°C, Zers.
MS(FAB,NBA): 270.2 (M + H)⁺.
R_{F}: 0.35 (Dichlormethan/Methanol/Triethylamin 8:1:1)

### Beispiel 21

### N-[2-(4-Methoxyphenyl-diphenylmethyl-amino)-6-hydroxy-purin-9-yl-acetyl]-N-2-(9-fluorenyl-methyloxycarbonylamino)-ethyl-glycin

### Fmoc-Aeg(G^{Mmt})-OH

1 g 2-(4-Methoxyphenyl-diphenylmethyl-amino)-6-hydroxy-purin-9-yl-essigsäure werden in DMF gelöst und zu der auf 0°C gekühlten Lösung werden nacheinander 682 mg TOTU und 239 mg N-Ethylmorpholin gegeben. Die Mischung wird noch 20 min bei Raumtemperatur nachgerührt. In einem separaten Kolben wird eine Lösung von 1.06 g Fmoc-aminoethylglycin in 4 ml Dimethylformamid und 1.5 g Bis-trimethylsilylacetamid bereitet. Diese Lösung wird dann zu der voraktivierten Lösung von 2-(4-Methoxyphenyldiphenylmethyl-amino)-6-hydroxy-purin-9-yl-essigsäure gegeben. Die Mischung wird weitere 2 h gerührt und dann zur Trockene eingeengt. Danach wird in 30 ml Ethylacetat aufgenommen und dreimal mit je 15 ml Wasser extrahiert. Die organische Phase wird über Natriumsulfat getrocknet und an Kieselgel mit Methanol/Dichlormethan/Wasser/Eisessig (1.5:10:0.25:0.075) chromatographisch gereinigt.
Ausbeute: 600 mg
R_{F}: 0.35 (Methylenchlorid:Methanol/7:3)
MS(FAB, DMSO/NBA/LiCl): 810.3(M+Li)⁺

### Beispiel 22

### N-[6-(4-Methoxyphenyl-diphenylmethyl-amino)-purin-9-yl-acetyl]-N-2-(9-fluorenyl-methyloxycarbonylamino)-ethyl-glycin-methylester

### Fmoc-Aeg(A^{Mmt})-OMe

Eine Mischung aus 848 mg (2.6 mmol) Fmoc-Aeg-OMe · HCI und 407 mg (2.5 mmol) HOOBt wird in 5 ml DMF gelöst, dann 640 µl (5 mmol) NEM und 1.2 g (2.5 mmol) 6-(4-Methoxyphenyl-diphenylmethyl-amino)-purin-9-yl-essigsäure gelöst in 5ml DMF dazugegeben. Danach werden 471 µl (3 mmol) Diisopropylcarbodiimid zugefügt und die Mischung 4 h bei Raumtemperatur gerührt. Dann wird das Gemisch filtriert und das Filtrat am Rotationsverdampfer im Vakuum eingeengt. Der amorphe Rückstand wird in Dichlormethan aufgenommen, mit Wasser und Natriumhydrogencarbonat-Lösung extrahiert, die organische Phase über Natriumsulfat getrocknet und dann im Vakuum zur Trockene eingeengt. Der Rückstand wird in 4.5 ml Ethylacetat gelöst und durch Zugabe von 20 ml Diisopropylether gefällt. Der daraus erhaltene halbfeste Niederschlag wird in 20 ml Methanol gelöst, durch Zugabe von 30 ml Wasser ausgefällt und dann im Vakuum getrocknet.
Ausbeute: 1.05 g
R_{F}: 0.85 (n-Butanol/Essigsäure/Wasser 3:1:1)
MS(FAB, DMSO/NBA):802.4(M+H)⁺

### Beispiel 23

### Synthese von N-[ 6-(4-Methoxyphenyl-diphenylmethyl-amino)-purin-9-yl-acetyl]-N-2-(9-fluorenyl-methyloxycarbonylamino)-ethyl-glycin

### Fmoc-Aeg(A^{Mmt})-OH

900 mg (1.125 mmol) Fmoc-Aeg(A^{Mmt})-OMe werden in einer Mischung aus 5 ml Dioxan und 2.5 ml Wasser gelöst und unter Kühlung mit Eiswasser durch portionsweise Zugabe einer Mischung aus 2.4 ml 1N NaOH und 2.5 ml Dioxan verseift. Nach beendeter Reaktion wird durch Zugabe von wenig festem Kohlendioxid abgepuffert und die in geringem Maße erfolgte Fmoc-Abspaltung durch Zugabe von Fmoc-ONSU rückgängig gemacht. Dann wird das Dioxan weitgehend in Vakuum abdestilliert, die Lösung mit Wasser verdünnt und mit Ethylacetat - n-Butanol (1:1) überschichtet. Das Gemisch wird durch Zugabe von Kaliumhydrogensulfat-Lösung unter Kühlung mit Eiswasser auf pH 5 bis 6 angesäuert. Die organische Phase wird abgetrennt und die wäßrige Phase noch zweimal mit Ethylacetat - Butanol (1:1) extrahiert. Die vereinigten organischen Phasen werden über Natriumsulfat getrocknet und am Rotationsverdampfer im Vakuum eingeengt. Der Rückstand wird in 5 ml Ethylacetat gelöst und durch Zugabe von 20 ml Methyl-butyl-ether ausgefällt.
Ausbeute: 1.02 g
R_{F}: 0.73 (n-Butanol/Essigsäure/Wasser 3:1:1)
MS (FAB, DMSO/NBA): 787 (M+H)⁺, 794 (M+Li)⁺

### Beispiel 24

### N-(2.4-Dihydroxy-5-methyl-pyrimidin-1-yl-acetyl)-N-2-(9-fluorenyl-methyloxy-carbonylamino)-ethyl-glycin

### Fmoc-Aeg(T)-OH

4.03 g Fmoc-Aeg-OH werden in 80 ml DMF suspendiert, mit 4.39 ml N,O-Bistrimethylsilylacetamid versetzt und 40 min bei Raumtemperatur gerührt. Zu der klaren Lösung werden dann bei 0°C 2.4 g 2.4-Dihydroxy-5-methyl-pyrimidin-essigsäurechlorid unter gutem Rühren gegeben. Man läßt noch 3 h bei Raumtemperatur nachreagieren und destilliert dann das Lösungsmittel am Rotationsverdampfer ab. Das so erhaltene Rohprodukt wird mit Wasser versetzt und über Nacht bei 0°C stehengelassen. Dann wird das Wasser abdekantiert, der Rückstand im Hochvakuum getrocknet und an Kieselgel mit Dichlormethan/Methanol/Eisessig/Wasser (500/100/25/2) chromatographisch gereinigt. Die vereinigten Produkt-Fraktionen werden zur Trockene eingeengt und noch dreimal mit wenig Toluol nachdestilliert.
Ausbeute: 3.8 g
R_{F}: 0.42(Dichlormethan/Methanol/Essigsäure 100:20:5)
MS(FAB, DMSO/NBA): 507.3(M+H)⁺

### Beispiel 25

### N-(2.4-Dihydroxy-5-methyl-pyrimidin-1-yl-acetyl)-N-2-(9-fluorenyl-methyloxy-carbonylamino)-ethyl-glycin

### Fmoc-Aeg(T)-OH

Eine Mischung aus 386 mg (2 mmol) 2.4-Dihydroxy-5-methyl-pyrimidin-essigsäure und 652 mg (4 mmol) HOOBt wird in 6 ml trockenem DMF kurz auf 70 bis 80°C erwärmt. Nach Abkühlen auf Raumtemperatur werden 314 µl (2 mmol) Diisopropylcarbodiimid zugefügt und die Mischung 30 min gerührt. Danach werden 680 mg (2 mmol) Fmoc-Aeg-OH zugegeben. In Abständen von 30 min wird das Gemisch dreimal kurz auf ca. 70°C erwärmt. Dann wird das Lösungsmittel am Rotationsverdampfer im Vakuum abgedampft und der verbliebene Rückstand mit Wasser verrieben und das so erhaltene Rohprodukt zwischen n-Butanol und Wasser verteilt. Die organische Phase wird mit Kaliumhydrogensulfat-Lösung, Natriumhydrogencarbonat-Lösung und Wasser extrahiert. Dann wird die organische Phase am Rotationsverdampfer im Vakuum eingeengt wobei das Produkt ausfällt.
Ausbeute: 260 mg
R_{F}: 0.40 (n-Butanol/Essigsäure/Wasser 3:1:1)
MS(FAB, DMSO/NBA): 507.3(M+H)⁺

### Beispiel 26

### N-[2-Hydroxy-4-(4-Methoxyphenyl-diphenylmethyl-amino)-pyrimidin-1-yl-acetyl]-N-2-(9-fluorenyl-methyloxycarbonylamino)-ethyl-glycin-methylester

### Fmoc-Aeg(C^{Mmt})-OMe

Eine Mischung aus 1.7 g (5 mmol) Fmoc-Aeg-OMe · HCl und 675 mg (5 mmol) HOOBt werden in 10ml getr. DMF gelöst und 1.28ml (10 mmol) NEM hinzugegeben. Dann wird die Mischung auf 0°C gekühlt, 2.21 g (5 mmol) 2-Hydroxy-4-(4-Methoxyphenyl-diphenylmethyl-amino)-pyrimidin-1-yl-essigsäure zugefügt gefolgt von 5 mmol Diisopropylcarbodiimid zugefügt und dieses Gemisch noch 30 min bei 0°C gerührt. Man rührt anschließend 4.5 h bei Raumtemperatur nach und destilliert dann das Lösungsmittel am Rotationsverdampfer im Vakuum ab. Der Rückstand wird zwischen Dichlormethan und Wasser verteilt, die organische Phase mit Natriumhydrogencarbonat-Lösung und Wasser gewaschen, über Natriumsulfat getrocknet und am Rotationsverdampfer im Vakuum zur Trockene eingeengt. Der Rückstand wird in 10 ml Methanol gelöst und durch Zugabe von 15 ml Wasser ausgefällt. Das erhaltene Produkt wird mit wenig Wasser verrieben und im Vakuumexsiccator über Kaliumhydroxid getrocknet.
Ausbeute: 3,19 g
R_{F}:0.79 (n-Butanol/Pyridin/Wasser/Essigsäure 8:2:2:10)
MS (FAB, DMSO/NBA): 778,4(M)⁺

### Beispiel 27

### N-[2-Hydroxy-4-(4-Methoxyphenyl-diphenylmethyl-amino)-pyrimidin-1-yl-acetyl]-N-2-(9-fluorenyl-methyloxycarbonylamino)-ethyl-glycin

### Fmoc-Aeg(C^{Mmt})-OH

3.0 g (3.86 mmol) Fmoc-Aeg(C^{Mmt})-OMe werden in einer Mischung aus 15 ml Dioxan und 10 ml Wasser gelöst und unter Kühlung mit Eiswasser durch portionsweise Zugabe von 10.2 ml einer Mischung aus 1N NaOH und Dioxan (1:1) verseift. Nach beendeter Reaktion wird durch Zufügen von wenig festem Kohlendioxid abgepuffert und die in geringem Maße erfolgte Fmoc-Abspaltung durch Zugabe von 0.5 g Fmoc-ONSU und 45-minütiges Nachrühren rückgängig gemacht. Dann wird die Mischung mit 0.5 ml 2M Kaliumhydrogensulfat-Lösung auf pH 6.5 gestellt. Anschließend wird das Dioxan weitgehend in Vakuum abdestilliert, die Lösung mit Wasser verdünnt und mit Ethylacetat überschichtet. Das Gemisch wird durch Zugabe von Kaliumhydrogensulfat-Lösung unter Kühlung mit Eiswasser auf pH 5 angesäuert. Die organische Phase wird abgetrennt und die wäßrige Phase noch zweimal mit Ethylacetat extrahiert. Die vereinigten organischen Phasen werden über Natriumsulfat getrocknet und am Rotationsverdampfer im Vakuum eingeengt. Der Rückstand wird in einer Mischung aus 5 ml Methanol und 15 ml Ethylacetat gelöst und durch Zugabe von 80 ml Methyl-butyl-ether ausgefällt. Das Produkt wird abfiltriert und im Vakuum getrocknet.
Ausbeute: 1.95 g
R_{F}: 0.80 (n-Butanol/Essigsäure/Wasser 3:1:1)
MS (FAB, DMSO/NBA/LiCl): 770,3 (M+Li)⁺

### Beispiel 28

### N-[2-Hydroxy-4-(tert.-butyloxycarbonyl-amino)-pyrimidin-1-yl-acetyl]-N-2-(9-fluorenyl-methyloxycarbonylamino)-ethyl-glycinmethylester

### Fmoc-Aeg(C^{Boc})-OMe

Eine Mischung aus 0.85 g (3 mmol) N⁴-(tert.Butyloxycarbonyl)-N¹-carboxymethyl cytosin und 0.49 g (3 mmol) HOOBt werden in 5 ml trockenem DMF gelöst, 786 µl (6 mmol) NEM zugegeben und dann 1.02 g (3mmol) Fmoc-Aeg-OMe · HCl zugefügt. Dann wird die Mischung auf 0°C gekühlt, 564 µl (3.6 mmol) Diisopropylcarbodiimid zugegeben und die Mischung 30 min bei 0°C und 4 h bei Raumtemperatur gerührt. Dann wird das Lösungsmittel am Rotationsverdampfer im Vakuum abdestilliert. Der Rückstand wird zwischen Dichlormethan und Wasser verteilt, die organische Phase mit Natriumhydrogencarbonat-Lösung und Wasser gewaschen, über Natriumsulfat getrocknet und am Rotationsverdampfer im Vakuum zur Trockene eingeengt. Der Rückstand wird aus 20 ml Ethylacetat umkristallisiert.
Ausbeute:1,19 g
R_{F}: 0.88 (n-Butanol/Essigsäure/Wasser 3:1:1)
MS (FAB, DMSO/NBA): 606.3 (M⁺)

### Beispiel 29

### Fmoc-Aeg(C^{Boc})-OMe

Eine Mischung aus 2.87 g (8.47 mmol) Fmoc-Aeg-OMe · HCI und 1.38 g (8.47 mmol) HOOBt werden in 15 ml trockenem DMF gelöst, 1.08 ml (8.47 mmol) NEM zugegeben und dann 2.4 g (8.47 mmol) N⁴-(tert.Butyloxycarbonyl)-N¹-carboxymethyl cytosin zugefügt. Dann wird die Mischung auf 0°C gekühlt, 1.33 ml (8.47 mmol) Diisopropylcarbodiimid zugegeben und die Mischung 30 min bei 0°C und 3 h bei Raumtemperatur gerührt. Dann wird das Lösungsmittel am Rotationsverdampfer im Vakuum abdestilliert. Der Rückstand wird in 100 ml Dichlormethan aufgenommen, die organische Phase nacheinander mit Wasser, Natriumhydrogencarbonat-Lösung und Wasser gewaschen, über Natriumsulfat getrocknet und am Rotationsverdampfer im Vakuum zur Trockene eingeengt. Der Rückstand wird Methyl-tert.-Butylether verrieben.
Ausbeute: 4.5g
R_{F}: 0.88 (n-Butanol/Essigsäure/Wasser 3:1:1)

### Beispiel 30

### N-[2-Hydroxy-4-(tert.-butyloxycarbonyl-amino)-pyrimidin-1-yl-acetyl]-N-2-(9-fluorenyl-methyloxycarbonylamino)-ethyl-glycin

### Fmoc-Aeg(C^{Boc})-OH

700 mg (1.15 mmol) Fmoc-Aeg(C^{Boc})-OMe werden in einer Mischung aus 6 ml Dioxan und 3 ml Wasser gelöst und bei 0°C portionsweise mit insgesamt 0.95 ml 2N NaOH innerhalb von 2 h versetzt. Nach beendeter Reaktion wird durch Zufügen von wenig festem Kohlendioxid abgepuffert und die in geringem Maße erfolgte Fmoc-Abspaltung durch Zugabe von 50 mg Fmoc-ONSU und 45-minütiges Nachrühren rückgängig gemacht. Dann wird die Mischung mit 0.5 ml 2M Kaliumhydrogensulfat-Lösung auf pH 6.5 gestellt. Anschließend wird das Dioxan weitgehend in Vakuum abdestilliert, die Lösung mit Wasser verdünnt und mit Ethylacetat überschichtet. Das Gemisch wird durch Zugabe von Kaliumhydrogensulfat-Lösung unter Kühlung mit Eiswasser auf pH 5 angesäuert. Die organische Phase wird abgetrennt und die wäßrige Phase noch zweimal mit Ethylacetat extrahiert. Die vereinigten organischen Phasen werden über Natriumsulfat getrocknet und am Rotationsverdampfer im Vakuum eingeengt wobei das Produkt ausfällt. Das Produkt wird abfiltriert und im Vakuum getrocknet.
Ausbeute: 485 mg
R_{F}: 0.55 (n-Butanol/Pyridin/Essigsäure/Wasser 8:2:2:10)
MS(FAB, DMSO/NBA): 592 (M⁺)

### Beispiel 31

### Fmoc-Aeg(T)-OMe

11.73 g Fmoc-Aeg-OMe · HCl werden zusammen mit 5.52 g Thyminylessigsäure in 100 ml trockenem DMF gelöst und dann nacheinander 9.84 g TOTU und 20.4 ml Diisopropylethylamin hinzugegeben. Die Mischung wird 3 h bei Raumtemperatur gerührt und dann im Vakuum am Rotationsverdampfer eingeengt. Der Rückstand wird in Ethylacetat aufgenommen und je dreimal mit Natriumhydrogencarbonat- und Kaliumhydrogensulfat-Lösung extrahiert. Die organische Phase wird eingengt, der Rückstand in wenig Ethylacetat gelöst und mit Ether versetzt wobei das Produkt ausfällt.
Ausbeute: 12.3 g
R_{F}: 0.63 (n-Butanol/Essigsäure/Wasser 3:1:1)
MS(ES⁺): 521.4(M+H)⁺

### Beispiel 32

### Fmoc-Aeg(T)-OH

12.0 g Fmoc-Aeg(T)-OMe werden in einer Mischung aus 100 ml Dioxan und 50 ml Wasser suspendiert durch portionsweise Zugabe von 32 ml 2N NaOH verseift. Nach beendeter Reaktion wird durch Zufügen von wenig festem Kohlendioxid abgepuffert und die teilweise erfolgte Fmoc-Abspaltung durch Zugabe von 2.7 g Fmoc-ONSU rückgängig gemacht. Dann wird die Mischung filtriert und das Filtrat mit Ethylacetat extrahiert. Die wäßrige Phase wird mit 1N HCI auf pH 2 gestellt wobei das Produkt ausfällt. Der Niederschlag wird abgesaugt und im Vakuum getrocknet (Rohprodukt 11.6 g). Zur Entfernung von Salzen wird das Rohprodukt in 70 ml DMF unter gelindem Erwärmen aufgenommen, Ungelöstes abfiltriert und das Filtrat zur Trockene eingeengt. Der Rückstand wird in Ethylacat aufgenommen und durch Zugabe von Diisopropylether ausgefällt. Das Produkt wird abgesaugt und im Vakuum getrocknet.
Ausbeute: 10.4 g
R_{F}: 0.40 (n-Butanol/Essigsäure/Wasser 3:1:1)
MS(ES⁺): 507.3 (M+H)⁺

### Beispiel 33

### 3-Benzyloxymethyl-2.4-dihydroxy-5-methyl-pyrimidin-essigsäure

### 3-Benyloxymethyl-thyminyl-essigsäure

13.65 g 3-Benzyloxymethyl-2.4-dihydroxy-5-methyl-pyrimidin-essigsäureethylester werden in einer Mischung aus 400 ml Dioxan, 200 ml Wasser, 100 ml Methanol und 40 ml 1N NaOH gelöst. Nach 2h Rühren bei Raumtemperatur ist die Verseifung beendet. Die Mischung wird im Vakuum am Rotationsverdampfer auf ein Volumen von ca. 200 ml eingeengt, mit 200 ml Wasser versetzt und einmal mit 100 ml Ethylacetat extrahiert (wird verworfen). Die wäßrige Phase wird mit 1N HCI auf pH 1.5 gestellt und mit dreimal 100 ml Ethylacetat extrahiert. Die Ethylacetatphasen werden vereinigt und über Natriumsulfat getrocknet. Nach Abfiltieren des Trockenmittels wird das Filtrat zur Trockene eingeengt. Es verbleibt ein zähes Öl, das direkt für die nächste Reaktion eingesetzt wird. Nach längerem Stehenlassen wird das Öl fest.
Ausbeute: 11.35 g
R_{F}: 0.64 (2-Butanon/Pyridin/Wasser/Essigsäure 70:15:15:2)
MS(ES⁺): 305.2 (M+H)⁺

### Beispiel 34

### N-(3-Benzyloxymethyl-2.4-dihydroxy-5-methyl-pyrimidin-1-yl-acetyl)-N-2-(9-fluorenyl-methyloxycarbonylamino)-ethyl-glycinmethylester

### Fmoc-Aeg(T^{Bom})-OMe

3.04 g 3-Benzyloxymethyl-2.4-dihydroxy-5-methyl-pyrimidin-essigsäure werden in 100 ml trockenem DMF gelöst und nacheinander 3.9 g Fmoc-Aeg-OMe · HCl, 3.28 g TOTU sowie 5.1 ml Diisopropylethylamin zugegeben. Die Mischung wird 2 h bei Raumtemperatur gerührt und dann im Vakuum am Rotationsverdampfer zur Trockene eingeengt. Der Rückstand wird in Ethylacetat aufgenommen und je dreimal mit Natriumhydrogencarbonat- und Kaliumhydrogensulfat-Lösung extrahiert. Die organische Phase wird über Natriumsulfat getrocknet und nach Abfiltrieren des Trockenmittels eingengt. Der verbleibende schaumige Rückstand wird mit Petrolether verrieben, abgesaugt und im Vakuum getrocknet.
Ausbeute: 4.68 g
R_{F}: 0.75 (n-Butanol/Essigsäure/Wasser 3:1:1)
MS(ES⁺): 641.4 (M+H)⁺

### Beispiel 35

### N-(3-Benzyloxymethyl-2.4-dihydroxy-5-methyl-pyrimidin-1-yl-acetyl)-N-2-(9-fluorenyl-methyloxycarbonylamino)-ethyl-glycin

### Fmoc-Aeg(T^{Bom})-OH

2.3 g Fmoc-Aeg(T^{Bom})-OMe werden in einer Mischung aus 30 ml Dioxan und 15 ml Wasser gelöst und durch portionsweise Zugabe von 7.36 ml 1N NaOH verseift. Nach beendeter Reaktion wird durch Zufügen von wenig festem Kohlendioxid abgepuffert und die teilweise erfolgte Fmoc-Abspaltung durch Zugabe von 0.41 g Fmoc-ONSU rückgängig gemacht. Dann wird die Mischung filtriert, das Dioxan im Vakuum am Rotationsverdampfer abdestilliert, mit 30 ml Wasser verdünnt und die noch alkalische Lösung einmal mit Ethylacetat extrahiert. Die wäßrige Phase wird mit 1N HCI auf pH 3 gestellt wobei das Produkt ausfällt. Nach Stehenlassen über Nacht im Kühlraum bei 4°C wird der Niederschlag abgesaugt und im Vakuum getrocknet. Aus dem Filtrat fällt nach dem Einengen auf kleineres Volumen weiteres Produkt aus.
Ausbeute: 1.47 g
R_{F}: 0.37 (n-Butanol/Essigsäure/Wasser 3:1:1)
MS(ES⁺): 627.2(M+H)⁺

### PNA-Synthesen:

### Beispiel 36

### H-(Aeg(T))₈-Lys-NH₂ (C₄₉ H₁₂₇ N₃₅ O₃₃, FG 2275,26)

Die Synthese erfolgt an einem Aminomethyl-Polystyrolharz mit 5-(Fmoc-Amino-4-methoxybenzyl)-2,4-dimethoxyphenylpropionsäure als Ankergruppe in einem Multiplen Peptidsynthesizer der Fa. Abimed.
Es werden 14.7 mg (10 µmol) Fmoc-Amidanker-Harz in DMF vorgequollen und in ein Reaktionsgefäß des Multiplen Peptidsynthesizers eingebracht. Man verwendet für die Synthese die folgenden Reaktionslösungen:

| | | |
|---|---|---|
| 1) | Aktivator-Lsg. | 0.876 molare PyBOP-Lösung in getrocknetem DMF |
| | | |
| 2) | Base für Aktivierung | 3.95 molare Lösung von NEM in getrocknetem DMF |
| | | |
| 3) | Fmoc-Lys(Boc)-OH | 0.65 molar in getrocknetem DMF |
| | | |
| 4) | Fmoc-Aeg(T)-OH | 250 mg in 1 ml getrocknetem DMF |
| | | |
| 5) | Piperidin | 20%ige Lösung in DMF. |

Nach Fmoc-Abspaltung mit Piperidin-Lösung und Waschen mit DMF werden Aktivatorlösung, Fmoc-Derivat und Base in das Reaktionsgefäß zum entschützten Harz gegeben. Die Kupplungszeit beträgt 40 min. Nach 28 min Reaktionszeit 100 µl Dichlormethan zugegeben. Anschließend wird das Harz 5mal mit DMF gewaschen und ist bereit für die nächste Piperidinbehandlung. Nach der letzten Fmoc-Abspaltung wird das Harz getrocknet und portionsweise mit insgesamt 4 ml 95%iger TFA behandelt (ca. 2.5 h). Die TFA-Lösung wird im Vakuum eingeengt und der Rückstand in 250 µl TFA gelöst. Diese Lösung wird auf 5 Eppendorf-Gefäße verteilt und mit je 600 µl Methyl-tert.-butyl-ether gefällt. Der Niederschlag wird durch Zentrifugieren sedimentiert und dann der Überstand abdekantiert. Der Niederschlag wird in wenig Methyl-tert.-butyl-ether suspendiert und erneut durch Zentrifugieren sedimentiert und dann wiederum der Überstand abdekantiert. Dieser Vorgang wird nochmals wiederholt und dann das Produkt im Vakuum getrocknet. Die Reinigung erfolgt durch Chromatographie über eine ®MonoQ-Säule (Fa. Pharmacia, Munzingen) mit 0.17 - 0.44 M Natriumchlorid-Gradienten in 10 mM NaOH (pH 12) und Entsalzung durch Ultrafiltration.
Ausbeute: 198 OD₂₆₀.
MS (FAB, AcOH/NBA): 2298 (M + Na)⁺

### Beispiel 37

### H-(Aeg(T^{triazolo})₈-Lys-NH₂ (C₁₁₀ H₁₃₄ N₅₈ O₂₆, FG 2682,65)

Die Synthese erfolgt an einem Aminomethyl-Polystyrolharz mit 5-(Fmoc-Amino-4-methoxybenzyl)-2.4-dimethoxyphenylpropionsäure als Ankergruppe mit einem ABI-DNA-Synthesizer 380B. Es werden 14.7mg (10 µmol) Fmoc-Amidanker-Harz in einem Synthesesäulchen durch Zugabe folgender Lösungen programmgesteuert umgesetzt:

| | | |
|---|---|---|
| Position 1 | Fmoc-Lys(Boc)-OH: | 0.66molare Lösung in DMF |
| Position 2 | Fmoc-Aeg(T^{triazolo})-OH | 735 mg in 2 ml getrocknetem DMF |
| Position 5 | Mischung aus 1.35 ml NEM und 1.7 ml DMF (3.46molar) | |
| Position 6 | PyBOP: | 0.91molare Lösung in getr. DMF |
| Position 14 | 20% Piperidin in DMF | |
| Position 16 | DMF zum Waschen des Harzes. | |
| Position 17 | DMF(getrocknet) für Reaktion | |

Die Aufgabemenge wird über die Flußrate auf einen 5-fachen Überschuß an Lysin- bzw. Aminoethyglycin-Derivat eingestellt.Die Kupplung erfolgt jeweils zweimal. Zur Abspaltung der Verbindung vom Harz werden insgesamt ca. 5 ml 90%ige TFA innerhalb 2h manuell duch das Säulchen gedrückt. Die TFA wird im Vakuum eingeengt, der Rückstand in 300 µl TFA gelöst und diese Lösung auf 6 Eppendorfgefäße verteilt. Das Produkt wird durch Zugabe von je 1 ml Methyl-tert.-butyl-ether gefällt. Der Niederschlag wird durch Zentrifugieren sedimentiert und dann der Überstand abdekantiert. Der Niederschlag wird in wenig Methyl-tert.-butyl-ether suspendiert und erneut durch Zentrifugieren sedimentiert und dann wiederum der Überstand abdekantiert. Dieser Vorgang wird nochmals wiederholt und dann das Produkt im Vakuum getrocknet. Am Ende wird nochmals in Methyl-tert.butyl-ether suspendiert, zentrifugiert und getrocknet.
Ausbeute: 270 OD₂₆₀.
MS (FAB, TFA/NBA): 2684 (M⁺)

### Beispiel 38

### H-(Aeg(C))₇-Lys-NH₂ (C₇₆ H₁₀₆ N₃₈ O₂₂, FG 1903,92)

Synthese wird wie in Beispiel 36 beschrieben durchgeführt, jedoch mit 750 mg Fmoc-Aeg(C^{Mmt})-OH, das in 1.5 ml getrocknetem DMF gelöst eingesetzt wird. Die Abspaltung und Fällung erfolgt wie oben beschrieben.
Ausbeute: 16 mg oder 218 OD₂₆₀
MS (FAB, TFA/NBA): 1905 (M + H)⁺

### Beispiel 39

### Ac-Aeg(A)-Aeg(C)-Aeg(A)-Aeg(T)-Aeg(C)-Aeg(A)-Aeg(T)-Aeg(G)-Aeg(G)-Aeg(T)-Aeg(C)-Aeg(G)-Lys-NH₂ (C₁₃₇H₁₇₆N₇₂O₃₈, FG 3439.39)

Die Synthese der PNA's erfolgt an einem Ecosyn D-300 DNA Synthesizer (Fa. Eppendorf/Biotronik, Maintal) mit 100 mg (5 µmol eines mit 5-(Fmoc-Amino-4-methoxybenzyl)-2,4-dimethoxyphenylpropionsäure als Ankergruppe beladenen Aminopropyl-CPG.

Für die Synthese wurden folgende Lösungen eingesetzt:

| | | |
|---|---|---|
| 1) | Aktivator-Lsg. | 0,3molare HATU-Lösung in getrocknetem DMF |
| | | |
| 2) | Base für Aktivierung | 0,3molare Lösung von NEM in getrocknetem DMF |
| | | |
| 3) | Fmoc-Lys(Boc)-OH | 0,3molare Lösung in DMF |
| | | |
| 4) | Abspaltung von Fmoc | 20 %ige Lösung von Piperidin in DMF |
| | | |
| 5) | Fmoc-Aeg(T)-OH | 0,3molare Lösung in getrocknetem DMF |
| | | |
| 6) | Fmoc-Aeg(A^{Mmt})-OH | 0,3molare Lösung in getrocknetem DMF |
| | | |
| 7) | Fmoc-Aeg(C^{Mmt})-OH | 0,3molare Lösung in getrocknetem DMF |
| | | |
| 8) | Fmoc-Aeg(G^{Mmt})-OH | 0,3molare Lösung in getrocknetem DMF |

Nach Fmoc-Abspaltung mit Piperidin-Lösung und Waschen mit DMF werden Aktivatorlösung, Fmoc-Derivat und Base in das Reaktionsgefäß zum entschützten Träger gegeben. Die Kupplungszeit beträgt 20 min. Anschließend wird das CPG 5 mal mit DMF gewaschen und ist bereit für die nächste Piperidinbehandlung. Nach Beendigung der Synthese wird der PNA-CPG-Träger getrocknet und wie oben beschrieben aufgearbeitet.
Ausbeute: 210 OD₂₆₀.
MS 3439.4 (ES⁺): (M)⁺

### Beispiel 40

### H-Asp-Aeg(C)-Aeg(C)-Aeg(A)-Aeg(T)-Aeg(G)-Aeg(G)-Aeg(T)-Aeg(C)-Aeg(C)-Aeg(C)-Asp-NH-(CH₂)₆-OH (C₁₁₉ H₁₅₇ N₅₇ O₃₈, FG 2993.94)

Die Synthese der PNA's erfolgt an einem Ecosyn D-300 DNA Synthesizer (Fa. Eppendorf/Biotronik, Maintal) mit 133 mg (5 µMol) eines mit 6-Methylaminohex-1-yl hemisuccinat beladenen Aminopropyl-CPG.

Für die Synthese wurden folgende Lösungen eingesetzt:

| | | |
|---|---|---|
| 1) | Aktivator-Lsg. | 0,3molare HATU-Lösung in getrocknetem DMF |
| | | |
| 2) | Base für Aktivierung | 0,3molare Lösung von NEM in getrocknetem DMF |
| | | |
| 3) | Fmoc-Asp(OtBu)-OH | 0,3molare Lösung in DMF |
| | | |
| 4) | Abspaltung von Fmoc | 20%ige Lösung von Piperidin in DMF |
| | | |
| 5) | Fmoc-Aeg(T)-OH | 0,3molare Lösung in getrocknetem DMF |
| | | |
| 6) | Fmoc-Aeg(A^{Mmt})-OH | 0,3molare Lösung in getrocknetem DMF |
| | | |
| 7) | Fmoc-Aeg(C^{Mmt})-OH | 0,3molare Lösung in getrocknetem DMF |
| | | |
| 8) | Fmoc-Aeg(G^{Mmt})-OH | 0,3molare Lösung in getrocknetem DMF |

Nach Fmoc-Abspaltung mit Piperidin-Lösung und Waschen mit DMF werden Aktivatorlösung, Fmoc-Derivat und Base in das Reaktionsgefäß zum entschützten Träger gegeben. Die Kupplungszeit beträgt 20 min. Anschließend wird das CPG 5 mal mit DMF gewaschen und ist bereit für die nächste Piperidinbehandlung. Nach Beendigung der Synthese wird der PNA-CPG-Träger getrocknet, zunächst mit 3%iger Trichloressigsäure die Mmt-Schutzgruppen der Basen und dann mit 60%iger TFA in Dichlormethan die tert.-Butylgruppen der Asparaginsäuren abgespalten. Durch Behandlung mit konz. Ammoniak-Lösung bei 65°C wird dann das PNA vom Träger gespalten und wie oben beschrieben aufgearbeitet.
Ausbeute: 100 OD₂₆₀.
MS 2994.4(ES⁺): (M+H)⁺

## Patentansprüche

1. Verfahren zur Herstellung von PNA-Oligomeren der Formel I 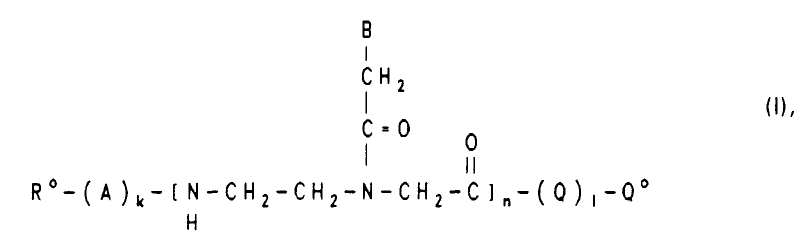 worin
R^{o} Wasserstoff, C₁-C₁₈-Alkanoyl, C₁-C₁₈-Alkoxycarbonyl, C₃-C₈-Cycloalkanoyl, C₇-C₁₅-Aroyl, C₃-C₁₃-Heteroaroyl, oder eine Gruppe bedeuten, die die intrazelluläre Aufnahme des Oligomers begünstigt oder die bei der Hybridisierung mit der target-Nucleinsäure interagiert;
A ein Aminosäurerest;
k eine ganze Zahl von Null bis 20;
Q ein Aminosäurerest;
I eine ganze Zahl von Null bis 20;
B für eine in der Nucleotidchemie übliche Nucleobase oder deren Prodrugformen;
Q^{o} Hydroxy, NH₂, NHR" bedeutet mit R" = C₁-C₁₈-Alkyl, C₂-C₁₈-Aminoalkyl, C₂-C₁₈-Hydroxyalkyl; und
n eine ganze Zahl von 1 - 50 bedeuten,
dadurch gekennzeichnet, daß man auf einen polymeren Träger der Formel II
L-[Polymer] (II),
der mit einer Ankergruppe L versehen ist, die latent den Rest Q^{o} enthält, entweder zuerst mit einem für die Festphasensynthese üblichen Verfahren Aminosäuren (Q') aufkuppelt und dann auf die dabei als Zwischenprodukt entstehende Verbindung der Formel III
(Q')ₗ-L-[Polymer] (III),
worin L wie oben definiert ist, Q' für eine Aminosäure Q steht, bei der die gegebenenfalls vorhandenen Seitenkettenfunktionen geschützt sind, und I für eine ganze Zahl von Null bis 20 steht,
oder direkt auf den polymeren Träger der Formel II
a) eine Verbindung der Formel IV 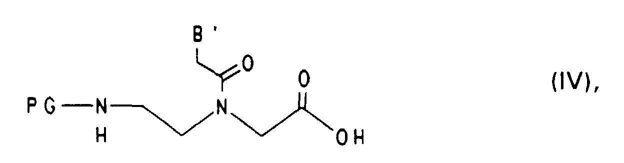 worin
PG für eine basenlabile Aminoschutzgruppe und
B' für eine Nucleobase stehen, deren exocyclische Aminofunktion durch eine mit der basenlabilen Aminoschutzgruppe Kompatiblen Schutzgruppe geschützt ist,
unter Verwendung der in der Peptidchemie üblichen Kupplungsreagenzien aufkuppelt,
b) die temporäre basenlabile Schutzgruppe PG mittels eines geeigneten Reagenzes abspaltet,
c) die Schritte a und b (n-1)fach wiederholt,
d) mit einem für die Festphasensynthese üblichen Verfahren weitere Aminosäuren A', die wie A definiert sind, jedoch in der Seitenkette gegebenfalls geschützt sind, aufkuppelt und dann für den Fall, daß R^{o} nicht Wasserstoff ist, den Rest R^{o} mit einem üblichen Verfahren einführt,
e) aus der als Zwischenverbindung erhaltenen Verbindung der Formel la 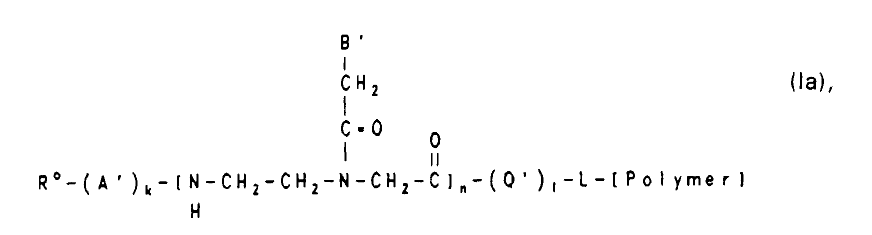 worin R^{o}, A', k, B', n, Q', und 1 wie oben definiert sind und L für eine Ankergruppe steht,
die Verbindung der Formel I mittels eines Abspaltungsreagenzes vom polymeren Träger abspaltet, wobei gleichzeitig oder auch anschließend die an der exocyclischen Aminofunktion der Nucleobasen und an den Seitenketten der Aminosäuren gegebenenfalls vorhandenen Schutzgruppen abgespalten werden.

2. Verfahren zur Herstellung von PNA-Oligomeren der Formel I gemäß Anspruch 1, dadurch gekennzeichnet, daß in Formel I
A für einen Aminosäurerest aus der Reihe Glycin, Leucin, Histidin, Phenylalanin, Cystein, Lysin, Arginin, Asparaginsäure, Glutaminsäure, Prolin, Tetrahydrochinolin-3-carbonsäure, Octahydroindol-2-carbonsäure und N-(2-Aminoethyl)glycin steht;
k eine ganze Zahl von Null bis 10 ist;
Q für einen Aminosäurerest aus der Reihe Glycin, Leucin, Histidin, Phenylalanin, Cystein, Lysin, Arginin, Asparaginsäure, Glutaminsäure, Prolin, Tetrahydrochinolin-3-carbonsäure, Octahydroindol-2-carbonsäure und N-(2-Aminoethyl)glycin steht;
I eine ganze Zahl von Null bis 10 ist;
B für eine natürliche Nucleobase aus der Reihe Adenin, Cytosin, Guanin, Thymin und Uracil oder eine unnatürliche Nucleobase aus der Reihe Purin, 2.6-Diaminopurin, 7-Deazaadenin, 7-Deazaguanin, N⁴N⁴-Ethanocytosin, N⁶N⁶-Ethano-2.6-diaminopurin, 5-Methylcytosin, 5-(C₃-C₆)-Alkinyl-uracil, 5-(C₃-C₆)-Alkinyl-cytosin, 5-Fluor-uracil, Pseudoisocytosin und 2-Hydroxy-5-methyl-4-triazolopyrimidin oder deren Prodrugformen steht; und
n eine ganze Zahl von 4 - 35 bedeutet.

3. Verbindungen der Formel IV 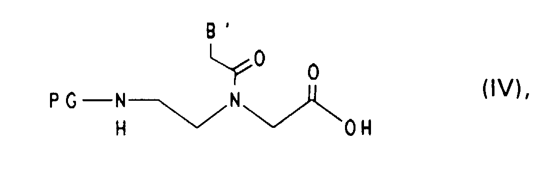 worin
PG für eine basenlabile Aminoschutzgruppe und
B' für eine Nucleobase stehen, deren exocyclische Aminofunktion durch eine mit der basenlabilen Aminoschutzgruppe kompatiblen Schutzgruppe geschützt ist.

4. Verbindung der Formel IV gemäß Anspruch 3, dadurch gekennzeichnet, daß
PG die Fmoc, Bnpeoc oder Dnpeoc-Schutzgruppe bedeutet und
B' für eine Nucleobase steht, deren exocyclische Aminofunktion durch eine gegen schwache oder mittelstarke Säuren labile Schutzgruppe vom Urethantyp oder Trityl-Typ geschützt ist.

5. Verbindung der Formel IV gemäß Anspruch 4, dadurch gekennzeichnet, daß
PG die Fmoc-Schutzgruppe und
B' für eine Nucleobase steht, deren exocyclische Aminofunktion durch eine gegen schwache oder mittelstarke Säuren labile Schutzgruppe vom Urethantyp oder Trityl-Typ geschützt ist.

6. Verfahren zur Herstellung von Verbindungen der Formel IV gemäß den Ansprüchen 3 bis 5, dadurch gekennzeichnet, daß man
a) eine Verbindung der Formel V 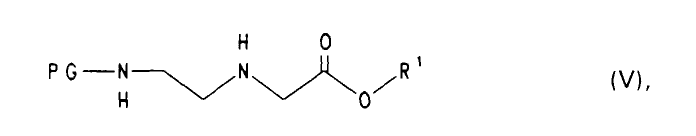 worin
PG eine basenlabile Aminoschutzgruppe und
R¹ eine Esterschutzgruppe bedeuten,
mit einer Verbindung der Formel VI 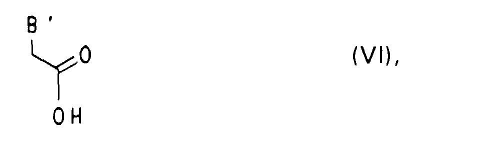 worin
B' eine Nucleobase, deren exocyclische Aminofunktion durch eine mit der basenlabilen Aminoschutzgruppe kompatiblen Schutzgruppe geschützt ist, bedeutet,
bei 0-45°C in einem geeigneten Lösungmittel oder Mischungen dieser Lösungsmittel unter Verwendung eines in der Peptidchemie üblichen Kupplungsreagenzes zu einer Verbindung der Formel VII 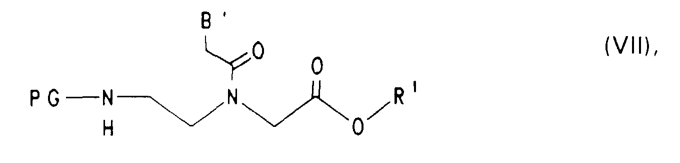 worin
PG , B' und R¹ wie oben definiert sind,
umsetzt, und anschließend die Esterschutzgruppe R¹ mit Alkalilauge oder auch enzymatisch mit Hilfe von Esterasen oder Lipasen bei 0-50°C in einem geeigneten Lösungsmittel oder Gemischen aus diesen Lösungsmitteln abspaltet, oder
b) eine Verbindung der Formel V 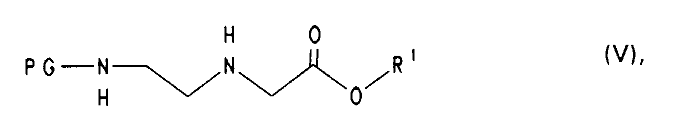 worin
PG eine basenlabile Aminoschutzgruppe in der obigen Bedeutung ist und
R¹ Wasserstoff oder eine temporäre Silylschutzgruppe bedeutet,
mit einer Verbindung der Formel VIII
B' - CH₂ - CO - R² (VIII),
worin
B' wie in Formel VI definiert ist, und
R² Halogen oder den Rest eines Aktivesters bedeutet,
bei 0-40°C in einem geeigneten Lösungsmittel oder Mischungen dieser Lösungsmittel umsetzt.

7. Verfahren zur Herstellung von Verbindungen der Formel IV gemäß Anspruch 6, dadurch gekennzeichnet, daß man
a) eine Verbindung der Formel V, worin
PG eine basenlabile Aminoschutzgruppe und
R¹ eine Esterschutzgruppe aus der Reihe Methyl, Ethyl, Butyl, 2-(Methoxyethoxy)-ethyl und Benzyl bedeuten,
mit einer Verbindung der Formel VI, worin
B' eine Nucleobase, deren exocyclische Aminofunktion durch eine gegen schwache oder mittelstarke Säuren labile Schutzgruppe vom Urethantyp oder Trityl-Typ geschützt ist, bedeutet,
bei Raumtemperatur in einem Lösungmittel aus der Reihe DMF, Acetonitril und Dichlormethan oder Mischungen dieser Lösungsmittel unter Verwendung von Carbodiimiden, Phosphonium-Reagenzien, Uronium-Reagenzien, Säurehalogeniden oder aktivierten Estern zu einer Verbindung der Formel VII, worin
PG, B' und R¹ wie oben definiert sind,
umsetzt, und anschließend die Esterschutzgruppe R¹ mit Alkalilauge oder auch enzymatisch mit Hilfe von Esterasen oder Lipasen bei 0-50°C in einem Lösungsmittel aus der Reihe Dioxan, Wasser, Tetrahydrofuran, Methanol und Wasser oder Gemischen aus diesen Lösungsmitteln abspaltet, oder
b) eine Verbindung der Formel V, worin
PG eine basenlabile Aminoschutzgruppe in der obigen Bedeutung ist und
R¹ Wasserstoff oder Trimethylsilyl bedeutet,
mit einer Verbindung der Formel VIII, worin
B' wie in Formel VI definiert ist und
R² Fluor, Chlor, Brom oder den Rest eines Aktivesters aus der Reihe OBt, OObt, OPfp und ONSu bedeuten,
bei 20 - 30°C in einem Lösungsmittel aus der Reihe DMF, NMP, Acetonitril und Dichlormethan oder Mischungen dieser Lösungsmittel umsetzt.

## Claims

1. A process for preparing PNA oligomers of the formula I 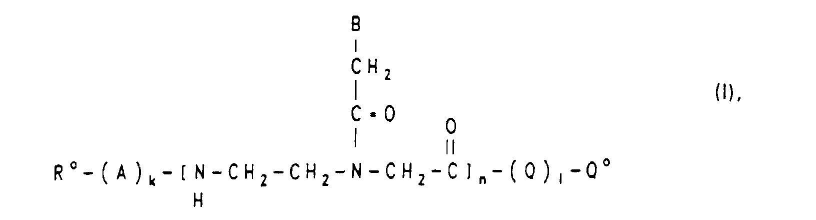 in which
R⁰ is hydrogen, C₁-C₁₈-alkanoyl, C₁-C₁₈-alkoxycarbonyl, C₃-C₈-cycloalkanoyl, C₇-C₁₅-aroyl, C₃-C₁₃-heteroaroyl, or a group which favors the intracellular uptake of the oligomer or which interacts with the target nucleic acid in the hybridization;
A is an amino acid residue;
k is an integer from zero to 20;
Q is an amino acid residue;
l is an integer from zero to 20;
B is a nucleobase which is customary in nucleotide chemistry, or its prodrug forms;
Q⁰ is hydroxyl, NH₂ or NHR", with R" = C₁-C₁₈-alkyl, C₂-C₁₈-aminoalkyl or C₂-C₁₈-hydroxyalkyl; and
n is an integer of 1-50,
wherein either amino acids (Q') are first coupled, using a method which is customary for solid phase synthesis, to a polymeric support of the formula II
L-[polymer] (II),
which is provided with an anchor group L which contains the radical Q⁰ in a latent manner, to result in, as an intermediate, a compound of the formula III
(Q')ₗ-L-[polymer] (III),
in which L is defined as above, Q' is an amino acid Q in which the side chain functions which may be present are protected, and 1 is an integer from zero to 20, and
a) a compound of the formula IV 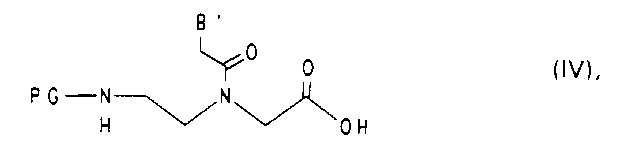 in which
PG is a base-labile amino protecting group, and
B' is a nucleobase whose exocyclic amino function is protected by a protecting group which is compatible with the base-labile amino protecting group,
is then coupled onto the compound of the formula III, or a compound of the formula IV is coupled directly to the polymeric support of the formula II, using the coupling reagents which are customary in peptide chemistry,
b) the temporary, base-labile protecting group PG is eliminated using a suitable reagent,
c) steps a and b are repeated n-1 times,
d) further amino acids A', which are defined as A but are, where appropriate, protected in the side chain, are coupled on using a method which is customary for solid phase synthesis and then, if R⁰ is not hydrogen, the radical R⁰ is introduced using a customary method,
e) the compound of the formula I is cleaved, out of the compound of the formula Ia 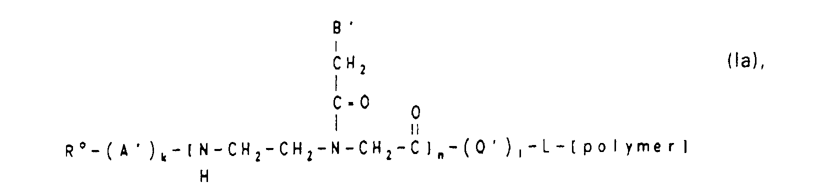
which is obtained as intermediate compound and in which R⁰, A', k, B', n, Q' and 1 are defined as above and L is an anchor group,
from the polymeric support using a cleaving reagent, with the protecting groups which are, where appropriate, present on the exocyclic amino function of the nucleobases and on the side chains of the amino acids being eliminated simultaneously or else subsequently.

2. The process for preparing PNA oligomers of the formula I as claimed in claim 1, wherein, in formula I,
A is an amino acid residue from the group glycine, leucine, histidine, phenylalanine, cysteine, lysine, arginine, aspartic acid, glutamic acid, proline, tetrahydroquinoline-3-carboxylic acid, octahydroindole-2-carboxylic acid and N-(2-aminoethyl)glycine;
k is an integer from zero to 10;
Q is an amino acid residue from the group glycine, leucine, histidine, phenylalanine, cysteine, lysine, arginine, aspartic acid, glutamic acid, proline, tetrahydroquinoline-3-carboxylic acid, octahydroindole-2-carboxylic acid and N-(2-aminoethyl)glycine;
l is an integer from zero to 10;
B is a natural nucleobase from the group adenine, cytosine, guanine, thymine and uracil, or an unnatural nucleobase from the group purine, 2,6-diaminopurine, 7-deazaadenine, 7-deazaguanine, N⁴,N⁴-ethanocytosine, N⁶,N⁶-ethano-2,6-diaminopurine, 5-methylcytosine, 5-(C₃-C₆)-alkynyl-uracil, 5- (C₃-C₆)-alkynyl-cytosine, 5-fluorouracil, pseudoisocytosine and 2-hydroxy-5-methyl-4-triazolopyrimidine, or their prodrug forms; and
n is an integer of 4-35.

3. A compound of the formula IV 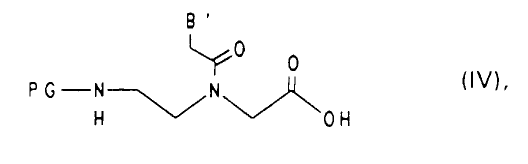 in which
PG is a base-labile amino protecting group, and
B' is a nucleobase whose exocyclic amino function is protected by a protecting group which is compatible with the base-labile amino protecting group.

4. A compound of the formula IV as claimed in claim 3, wherein
PG is the Fmoc, Bnpeoc or Dnpeoc protecting group, and
B' is a nucleobase whose exocyclic amino function is protected by a protecting group which is labile to weak or medium-strength acids and which is of the urethane type or trityl type.

5. A compound of the formula IV as claimed in claim 4, wherein
PG is the Fmoc protecting group, and
B' is a nucleobase whose exocyclic amino function is protected by a protecting group which is labile to weak or medium-strength acids and which is of the urethane type or trityl type.

6. A process for preparing a compound of the formula IV as claimed in claims 3 to 5, wherein
a) a compound of the formula V 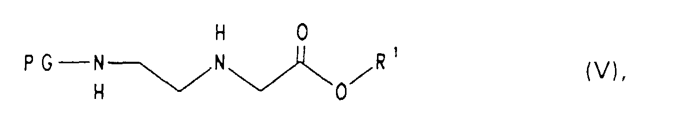 in which
PG is a base-labile amino protecting group, and
R¹ is an ester protecting group,
is reacted with a compound of the formula VI 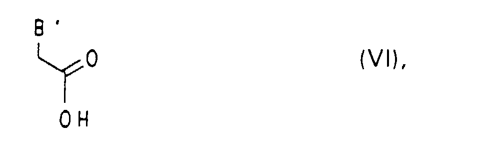 in which
B' is a nucleobase whose exocyclic amino function is protected by a protecting group which is compatible with the base-labile amino protecting group,
at 0-45°C in a suitable solvent, or mixtures of these solvents, using a coupling reagent which is customary in peptide chemistry, to yield a compound of the formula VII 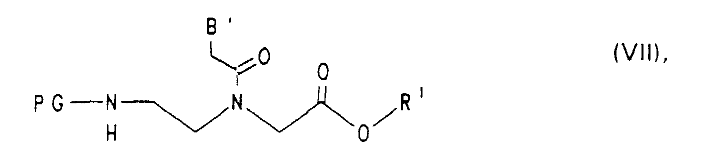 in which
PG, B' and R¹ are defined as above,
and the ester protecting group R¹ is then eliminated with alkali metal hydroxide solution or else enzymically with the aid of esterases or lipases at 0-50°C in a suitable solvent, or mixtures of these solvents, or
b) a compound of the formula V 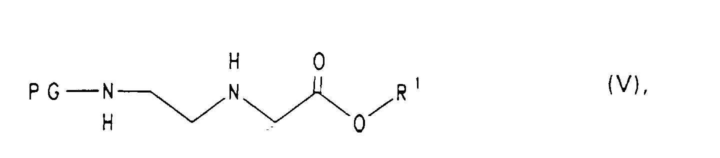 in which
PG is a base-labile amino protecting group having the above meaning, and
R¹ is hydrogen or a temporary silyl protecting group,
is reacted with a compound of the formula VIII
B'-CH₂-CO-R² (VIII),
in which
B' is defined as in formula VI, and
R² is halogen or the radical of an active ester,
at 0-40°C in a suitable solvent, or mixtures of these solvents.

7. The process for preparing a compound of the formula IV as claimed in claim 6, wherein
a) a compound of the formula V, in which
PG is a base-labile amino protecting group, and
R¹ is an ester protecting group from the group methyl, ethyl, butyl, 2-(methoxyethoxy)ethyl and benzyl,
is reacted with a compound of the formula VI, in which
B' is a nucleobase whose exocyclic amino function is protected by a protecting group which is labile to weak or medium-strength acids and which is of the urethane type or trityl type,
at room temperature in a solvent from the group DMF, acetonitrile and dichloromethane, or mixtures of these solvents, using carbodiimides, phosphonium reagents, uronium reagents, acid halides or activated esters, to yield a compound of the formula VII, in which
PG, B' and R' are defined as above,
and the ester protecting group R¹ is then eliminated with alkali metal hydroxide solution or else enzymically with the aid of esterases or lipases at 0-50°C in a solvent from the group dioxane, water, tetrahydrofuran, methanol and water, or mixtures of these solvents, or
b) a compound of the formula V, in which
PG is a base-labile amino protecting group having the above meaning, and
R¹ is hydrogen or trimethyl silyl,
is reacted with a compound of the formula VIII, in which
B' is defined as in formula VI, and
R² is fluorine, chlorine, bromine or the radical of an active ester from the group OBt, OObt, OPfp and ONSu,
at 20-30°C in a solvent from the group DMF, NMP, acetonitrile and dichloromethane, or mixtures of these solvents.

## Revendications

1. Procédé pour la préparation d'oligomères de PNA de formule I 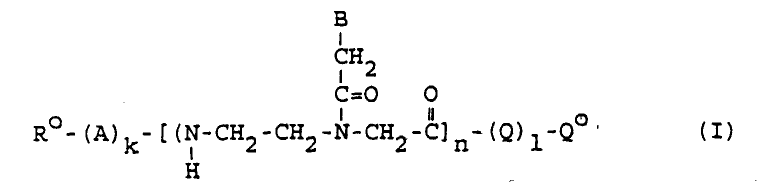 dans laquelle
R^{o} représente un atome d'hydrogène ou un groupe alcanoyle en C₁-C₁₈, alcoxy(C₁-C₁₈)carbonyle, cycloalcanoyle en C₃-C₈, aroyle en C₇-C₁₅, hétéroaroyle en C₃-C₁₃ ou un groupe qui facilite la capture intracellulaire de l'oligomère ou qui entre en interaction lors de l'hybridation avec l'acide nucléique cible;
A représente un reste d'aminoacide;
k est un nombre entier allant de zéro à 20;
Q représente un reste d'aminoacide;
l est un nombre entier allant de zéro à 20;
B représente une base nucléique usuelle dans la chimie des nucléotides ou des précurseurs de celle-ci;
Q^{o} représente un groupe hydroxyle, NH₂, NHR", R" étant un groupe alkyle en C₁-C₁₈, aminoalkyle en C₂-C₁₈ ou hydroxyalkyle en C₂-C₁₈; et
n est un nombre entier allant de 1 à 50;
caractérisé en ce que, sur un support polymère de formule II
L-[polymère] (II)
qui est muni d'un groupe d'ancrage L qui contient de façon latente le radical Q^{o}, soit on attache d'abord des aminoacides (Q') à l'aide d'un procédé usuel pour la synthèse en phase solide, et ensuite, sur le composé de formule III ainsi obtenu en tant que produit intermédiaire
(Q')ₗ-L-[polymère] (III)
dans laquelle L est tel que défini plus haut, Q' représente un aminoacide Q dans lequel les fonctions de chaîne latérale éventuellement présentes sont protégées et 1 représente un nombre entier allant de zéro à 20,
soit directement sur le support polymère de formule II,
a) on attache un composé de formule IV 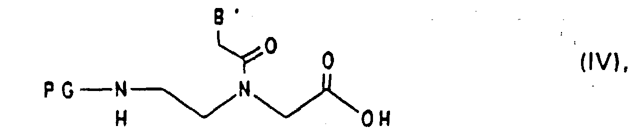 dans laquelle
PG représente un groupe protecteur de fonction amino labile en présence de bases et
B' représente une base nucléique dont la fonction amino exocyclique est protégée par un groupe protecteur compatible avec le groupe protecteur de fonction amino labile en présence de bases,
avec utilisation des réactifs de couplage usuels dans la chimie des peptides,
b) on élimine à l'aide d'un réactif approprié le groupe protecteur PG temporaire, labile en présence de bases,
c) on répète (n-1) fois les étapes a et b,
d) on attache, à l'aide d'un procédé usuel dans la synthèse en phase solide, d'autres aminoacides A' qui sont définis comme A mais sont éventuellement protégés sur la chaîne latérale, et ensuite, dans le cas où R^{o} n'est pas un atome d'hydrogène, on introduit le radical R^{o} à l'aide d'un procédé usuel,
e) à partir du composé de formule la 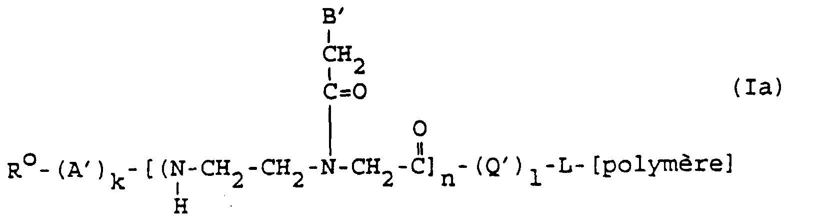 dans laquelle R^{o}, A', k, B', n, Q' et l sont tels que définis plus haut et L représente un groupe d'ancrage, obtenu en tant que composé intermédiaire,
on sépare le composé de formule I du support polymère au moyen d'un réactif de séparation, en éliminant en même temps ou bien ensuite les groupes protecteurs éventuellement présents sur la fonction amino exocyclique des bases nucléiques et sur les chaînes latérales des aminoacides.

2. Procédé pour la préparation d'oligomères de PNA de formule I selon la revendication 1, caractérisé en ce que, dans la formule I
A représente un reste d'aminoacide, choisi parmi la glycine, la leucine, l'histidine, la phénylalanine, la cystéine, la lysine, l'arginine, l'acide aspartique, l'acide glutamique, la proline, l'acide tétrahydroquinoléine-3-carboxylique, l'acide octahydro-indole-2-carboxylique et la N-(2-aminoéthyl)glycine;
k est un nombre entier allant de zéro à 10;
Q représente un reste d'aminoacide choisi parmi la glycine, la leucine, l'histidine, la phénylalanine, la cystéine, la lysine, l'arginine, l'acide aspartique, l'acide glutamique, la proline, l'acide tétrahydroquinoléine-3-carboxylique, l'acide octahydro-indole-2-carboxylique et la N-(2-aminoéthyl)glycine;
l est un nombre entier allant de zéro à 10;
B représente une base nucléique naturelle choisie parmi l'adénine, la cytosine, la guanine, la thymine et l'uracile, ou une base nucléique non naturelle, choisie parmi la purine, la 2,6-diaminopurine, la 7-déazaadénine, la 7-déazaguanine, la N⁴N⁴-éthanocytosine, la N⁶N⁶-éthano-2,6-diaminopurine, la 5-méthylcytosine, un 5-alcynyl(C₃-C₆)uracile, une 5-alcynyl(C₃-C₆)cytosine, le 5-fluoro-uracile, la pseudo-isocytosine et la 2-hydroxy-5-méthyl-4-triazolopyrimidine ou leurs précurseurs; et
n est un nombre entier allant de 4 à 35.

3. Composé de formule IV 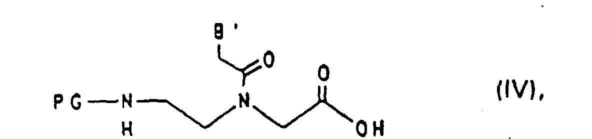 dans laquelle
PG représente un groupe protecteur de fonction amino labile en présence de bases et
B' représente une base nucléique dont la fonction amino exocyclique est protégée par un groupe protecteur compatible avec le groupe protecteur de fonction amino labile en présence de bases.

4. Composé de formule IV selon la revendication 3, caractérisé en ce que
PG représente le groupe protecteur Fmoc, Bnpeoc ou Dnpeoc, et
B' représente une base nucléique dont la fonction amino exocyclique est protégée par un groupe protecteur, labile en présence d'acides faibles ou moyennement forts, du type uréthanne ou du type trityle.

5. Composé de formule IV selon la revendication 4, caractérisé en ce que
PG représente le groupe protecteur Fmoc et
B' représente une base nucléique dont la fonction amino exocyclique est protégée par un groupe protecteur, labile en présence d'acides faibles ou moyennement forts, du type uréthanne ou du type trityle.

6. Procédé pour la préparation des composés de formule IV selon les revendications 3 à 5, caractérisé en ce que
a) on fait réagir un composé de formule V 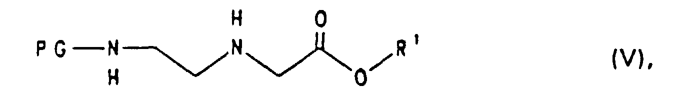 dans laquelle
PG représente un groupe protecteur de fonction amino labile en présence de bases et
R¹ représente un groupe protecteur estérifiant,
avec un composé de formule VI 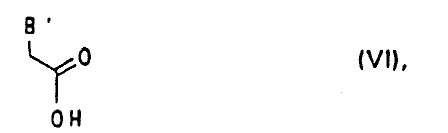 dans laquelle
B' représente une base nucléique dont la fonction amino exocyclique est protégée par un groupe protecteur compatible avec le groupe protecteur de fonction amino labile en présence de bases,
à 0-45°C, dans un solvant convenable ou des mélanges de ces solvants, avec utilisation d'un réactif de couplage usuel dans la chimie des peptides, pour l'obtention d'un composé de formule VII 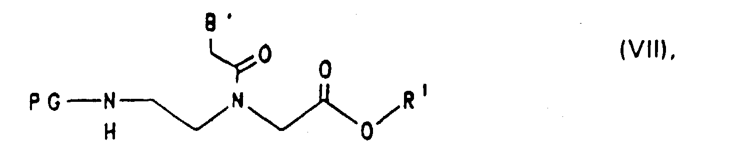 dans laquelle PG, B' et R¹ sont tels que définis plus haut,
et on élimine ensuite le groupe protecteur estérifiant R¹ à l'aide d'une solution d'un hydroxyde alcalin, ou bien par voie enzymatique à l'aide d'estérases ou de lipases, à 0-50°C, dans un solvant convenable ou des mélanges de tels solvants, ou
b) on fait réagir un composé de formule V 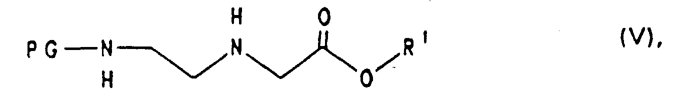 dans laquelle
PG représente un groupe protecteur de fonction amino labile en présence de bases, tel que défini plus haut, et
R¹ représente un atome d'hydrogène ou un groupe protecteur temporaire de type silyle,
avec un composé de formule VIII
B'-CH₂-CO-R² (VIII)
dans laquelle
B' est tel que défini dans la formule VI et
R² représente un atome d'halogène ou le reste d'un ester actif,
à 0-40°C, dans un solvant convenable ou des mélanges de tels solvants.

7. Procédé pour la préparation des composés de formule IV selon la revendication 6, caractérisé en ce que
a) on fait réagir un composé de formule V dans lequel
PG représente un groupe protecteur de fonction amino labile en présence de bases et
R¹ représente un groupe protecteur estérifiant choisi parmi les groupes méthyle, éthyle, butyle, 2-(méthoxyéthoxy)éthyle et benzyle,
avec un composé de formule VI, dans lequel
B' représente une base nucléique dont la fonction amino exocyclique est protégée par un groupe protecteur, labile en présence d'acides faibles ou moyennement forts, du type uréthanne ou du type trityle,
à la température ambiante, dans un solvant choisi parmi le DMF, l'acétonitrile et le dichlorométhane ou des mélanges de ces solvants, avec utilisation de carbodiimides, de réactifs à base de phosphonium, de réactifs à base d'uronium, d'halogénures d'acides ou d'esters activés, pour l'obtention d'un composé de formule VII dans lequel PG, B' et R¹ sont tels que définis plus haut,
et ensuite on élimine le groupe protecteur estérifiant R¹ à l'aide d'une solution d'un hydroxyde alcalin ou bien par voie enzymatique à l'aide d'estérases ou de lipases, à 0-50°C, dans un solvant choisi parmi le dioxanne, le tétra-hydrofuranne, le méthanol et l'eau, ou des mélanges de ces solvants, ou
b) on fait réagir un composé de formule V dans lequel
PG est un groupe protecteur de fonction amino labile en présence de bases, tel que défini plus haut, et
R¹ représente un atome d'hydrogène ou le groupe triméthylsilyle,
avec un composé de formule VIII dans lequel
B' est tel que défini dans la formule VI et
R² représente un atome de fluor, chlore ou brome ou le reste d'un ester actif choisi parmi OBt, OOBt, OPfp et ONSu,
à 20-30°C dans un solvant choisi parmi le DMF, la NMP, l'acétonitrile et le dichlorométhane ou des mélanges de ces solvants.
